# EUROPEAN PATENT APPLICATION

(11) **EP 4 129 324 A1**
(43) Date of publication of application: **08.02.2023**
(21) Application number: 21189218.7
(22) Date of filing: 02.08.2021
(51) Int. Cl.: A61K 38/26, A61K 38/22, A61P 3/10, A61P 3/04

(54) **COMPOSITIONS COMPRISING AT LEAST AN AMYLIN RECEPTOR AGONIST AND A GLP-1 RECEPTOR AGONIST**

(71) Applicant: Adocia, 69003 Lyon (FR)
(72) Inventor: The designation of the inventor has not yet been filed
(74) Representative: Tripoz, Inès

(57) **Abstract**

The invention relates to a method for the treatment of obesity, overweight, metabolic diseases and/or diabetes, in particular type 2 diabetes, wherein at least one short acting amylin RA that and at least one short acting GLP-1 RA are co-administered in a therapeutical scheme that comprises simultaneous or sequential administration.

It also relates to compositions, at least one of these comprising a short acting amylin RA and doesn't comprise any GLP-1 RA and at least one of these comprising a short acting GLP-1 RA and does not comprise an amylin RA for use for the treatment of obesity, overweight, metabolic diseases and/or diabetes, in particular type 2 diabetes, characterized in that the compositions are co-administered in a therapeutical scheme that comprises simultaneous or sequential administration.

It also relates to a composition containing at least one short acting amylin RA in combination with at least one short acting GLP-1 RA for use for treating obesity, overweight, metabolic diseases and/or diabetes, in particular type 2 diabetes.

## Description

The invention relates to therapies for treating obesity, overweight, metabolic diseases and/or diabetes, in particular type 2 diabetes.

In recent times, obesity has spread rapidly in the world with the US and China being the most affected population. Today, 650 million of adults (13% of adults) and about 124 million of children older than 5 are obese in the world. In the US, 42.8% of adults and 18.5% of children are obese.

It has major health implications that can cause premature death due the increased risk of developing several diseases including type II diabetes, cerebrovascular disease, sleep apnea, cancers.

A weight loss of 10 to 15% is thought to significantly reduce the incidence of severe complications.

To treat this disease, therapies have focused on reducing food intake in patients.

Diet and physical activity are the foundations for treating obesity, but the use of medications is becoming a prevalent strategy for a long-term success over this disease.

Amylin receptor agonists (amylin RA) are useful to reduce food intake and treating obesity.

Amylin receptor agonists and more particularly pramlintide, a human amylin analog, have been identified to diminish the food intake but it slows down gastric emptying.

GLP-1 receptor agonist (GLP-1 RA), also known as incretin mimetics, are insulin secretagogues. Among GLP-1 RA can be cited exenatide and lixisenatide, which are also known for diminishing the food intake and for slowing down the gastric emptying.

Amylin receptor agonists, and more particularly pramlintide, and GLP-1 RA, such as exenatide and lixisenatide, have several side effects, for example nausea, which may cause a problem of tolerability.

Native human amylin is a 37-amino acid peptide having the formula Hy-KC()NTATC()ATQRLANFLVHSSNNFGAILSSTNVGSNTY-NH₂ (SEQ ID NO: 1) wherein Hyat the N-terminus designates a hydrogen atom, corresponding to the presence of a free amino group on the N-terminal amino acid residue [i.e. the lysine (K) residue at sequence position number 1 in SEQ ID NO: 1]; wherein -NH₂ at the C-terminus indicates that the C-terminal carboxyl group is in the amide form; and wherein the parentheses () associated with the two cysteine (C, Cys) residues at sequence positions 2 and 7 indicate the presence of an intramolecular disulfide bridge between the two Cys residues in question.

Pramlintide is an amylin receptor agonist having the following formula Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Pro-lle-Leu-Pro-Pro-Thr-Asn-Val-Gly-Ser- Asn-Thr-Tyr (SEQ ID NO: 2)

Surprisingly, the applicant has demonstrated that a composition containing a short acting amylin RA, in combination with a short acting GLP-1 RA, such as exenatide or lixisenatide, allows reducing the slow-down of gastric emptying in comparison to an identical composition without exenatide or lixisenatide. These compositions may thus exhibit a better tolerability profile and a better compliance of the patients.

The invention relates to a composition comprising at least one amylin receptor agonist, and at least one short acting GLP-1 receptor agonist such as exenatide and lixisenatide.

The invention also relates to a composition containing at least one short acting amylin RA in combination with at least one short acting GLP-1 RA for use to reduce the slow-down of gastric emptying.

The invention also relates to a composition containing at least one short acting amylin RA in combination with at least one short acting GLP-1 RA for use for the treatment of obesity.

The invention also relates to a composition containing at least one short acting amylin RA in combination with at least one short acting GLP-1 RA for use for the treatment of metabolic diseases

The invention also relates to a composition containing at least one short acting amylin RA in combination with at least one short acting GLP-1 RA for use for treating obesity, overweight, metabolic diseases and/or diabetes, in particular type 2 diabetes.

In an embodiment, the composition containing a short acting amylin RA, in combination with a short acting GLP-1 RA is characterized in that the short acting amylin RA is not native human amylin or pramlintide.

In an embodiment, the composition containing a short acting amylin RA, in combination with a short acting GLP-1 RA is characterized in that the short acting amylin RA is not native human amylin.

In an embodiment, the composition containing a short acting amylin RA, in combination with a short acting GLP-1 RA is characterized in that the short acting amylin RA is not pramlintide.

In an embodiment, the composition containing a short acting amylin RA in combination with a short acting GLP-1 RA is characterized in that the short acting GLP-1 RA is exenatide or lixisenatide.

The invention also concerns a composition comprising a short acting amylin RA and a short acting GLP-1 receptor agonist (GLP-1 RA) as only pharmaceutical active ingredients (API).

The invention also concerns at least two compositions, at least one of these comprising a short acting amylin RA and doesn't comprise any GLP-1 RA and at least one of these comprising a short acting GLP-1 RA and does not comprise an amylin RA, for use to reduce the slow-down of gastric emptying, characterized in that the compositions are co-administered in a therapeutical scheme that comprises simultaneous or sequential administration.

The invention also concerns at least two compositions, at least one of these comprising a short acting amylin RA and doesn't comprise any GLP-1 RA and at least one of these comprising a short acting GLP-1 RA that does not comprise an amylin RA for use for the treatment of obesity, characterized in that the compositions are co-administered in a therapeutical scheme that comprises simultaneous or sequential administration.

The invention also concerns at least two compositions, at least one of these comprising a short acting amylin RA and doesn't comprise any GLP-1 RA and at least one of these comprising a short acting GLP-1 RA that does not comprise an amylin RA for use for the treatment of metabolic diseases, characterized in that the compositions are co-administered in a therapeutical scheme that comprises simultaneous or sequential administration.

The invention also concerns at least two compositions, at least one of these comprising a short acting amylin RA and doesn't comprise any GLP-1 RA and at least one of these comprising a short acting GLP-1 RA that does not comprise an amylin RA for use for the treatment of obesity, overweight, metabolic diseases and/or diabetes, in particular type 2 diabetes, characterized in that the compositions are co-administered in a therapeutical scheme that comprises simultaneous or sequential administration.

In an embodiment, the composition containing a short acting amylin RA, is characterized in that the short acting amylin RA is not native human amylin.

In an embodiment, the composition containing a short acting amylin RA, is characterized in that the short acting amylin RA is not pramlintide.

In an embodiment, the composition containing a short acting GLP-1 RA is characterized in that the short acting GLP-1 RA is exenatide or lixisenatide.

The invention also relates to a method for reducing the slow-down of gastric emptying, wherein at least one short acting amylin RA and at least one short acting GLP-1 are co-administered in a therapeutical scheme that comprises simultaneous or sequential administration.

The invention also relates to a method for the treatment of obesity, wherein at least one short acting amylin RA and at least one short acting GLP-1 RA are co-administered in a therapeutical scheme that comprises simultaneous or sequential administration.

The invention also relates to a method for the treatment of metabolic diseases, wherein at least one short acting amylin RA and at least one short acting GLP-1 RA are co-administered in a therapeutical scheme that comprises simultaneous or sequential administration.

The invention also relates to a method for the treatment of obesity, overweight, metabolic diseases and/or diabetes, in particular type 2 diabetes, wherein at least one short acting amylin RA that and at least one short acting GLP-1 RA are co-administered in a therapeutical scheme that comprises simultaneous or sequential administration.

The invention also relates to a method for reducing the slow-down of gastric emptying, wherein at least two compositions comprising at least one short acting amylin RA that does not comprise a GLP-1 RA and at least one short acting GLP-1 that does not comprise an amylin RA are co-administered in a therapeutical scheme that comprises simultaneous or sequential administration.

The invention also relates to a method for the treatment of obesity, wherein at least two compositions comprising at least one short acting amylin RA that does not comprise a GLP-1 RA and at least one short acting GLP-1 RA that does not comprise an amylin RA are co-administered in a therapeutical scheme that comprises simultaneous or sequential administration.

The invention also relates to a method for the treatment of metabolic diseases, wherein at least two compositions comprising at least one short acting amylin RA that does not comprise a GLP-1 RA and at least one short acting GLP-1 RA that does not comprise an amylin RA are co-administered in a therapeutical scheme that comprises simultaneous or sequential administration.

The invention also relates to a method for the treatment of obesity, overweight, metabolic diseases and/or diabetes, in particular type 2 diabetes, wherein at least two compositions comprising at least one short acting amylin RA that does not comprise a GLP-1 RA and at least one short acting GLP-1 RA that does not comprise an amylin RA are co-administered in a therapeutical scheme that comprises simultaneous or sequential administration.

In an embodiment the method is characterized in that the short acting amylin RA is not native human amylin.

In an embodiment the method is characterized in that the short acting amylin RA is not pramlintide

In an embodiment the method is characterized in that the short acting GLP-1 RA is exenatide or lixisenatide.

By "short-acting" amylin RA is meant an amylin RA that has an apparent half-life of elimination after subcutaneous injection in humans of less than 3 hours, in particular less than 2 hours, preferably less than 1.5 hours or even less than 1 hour.

By "short-acting" GLP-1 RA is meant a GLP-1 RA that has an apparent half-life of elimination after subcutaneous injection in humans of less than 8 hours, in particular less than 5 hours, preferably less than 4 hours or even less than 3 hours.

According to the invention, the short acting amylin RA is chosen amongst the peptides according to Formula 1:

**R₁-Z-R₂** Formula 1

wherein
- R₁ is chosen in the group consisting of hydrogen, C₁₋₄ acyl, benzoyl or C₁₋₄ alkyl;
- R₂ is OH or NHR₃, wherein R₃ is hydrogen or C₁₋₃-alkyl; and
- Z is an amino acid sequence according to formula A1 or B1 or C1 or D1 or E1, and

Formula A1 is the following amino acid sequence as set forth in (SEQ ID NO:3):
Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Xaa25-Xaa26-Xaa27-Xaa28-Xaa29-Thr-Xaa31-Val-Gly-Xaa34-Xaa35-Thr-Xaa37 (SEQ ID NO:3) wherein Xaa represents an amino acid and
wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Asp, Glu, His, Asn, Arg, Gly, Ala, Ser, Lys, Thr and Cys
- Xaa17 is independently selected from His, Arg, Lys and Val;
- Xaa18 is independently selected from Arg, Lys and His;
- Xaa21 is independently selected from Ala, Lys, Gln, Ser, Asn and Pro;
- Xaa22 is independently selected from Glu, Gln, Ser, Thr and Asn;
- Xaa25 is independently selected from Pro and Ala
- Xaa26 is independently selected from Pro, Arg and Ile;
- Xaa27 is independently selected from Pro and Leu;
- Xaa28 is independently selected from Pro and Ser
- Xaa29 is independently selected from Pro and Ser
- Xaa31 is independently selected from Ser, Glu, Asp, Pro and Asn;
- Xaa34 is independently selected from Pro, His, Lys, Arg and Ser;
- Xaa35 is independently selected from His, Arg, Lys, Asp, Glu and Asn;
- Xaa37 is independently selected from Pro and Tyr;

Formula B1 is the following amino acid sequence as set forth in (SEQ ID NO:4): 
X(-1)-X1-X2-X3-X4-X5-X6-X7-X8-X9-X10-X11-X12-X13-X14-X15-X16-X17-X18-X19-X20-X21-
X22-X23-X24-X25-X26-X27-X28-X29-X30-X31-X32-X33-X34-X35-X36 (SEQ IDNO: 4)
 wherein X represents an amino acid and
wherein
- X(-1) is absent or E,
- X1 is absent or selected from the group consisting of E or A,
- X2 is selected from the group consisting of L, A or P,
- X3 is selected from the group consisting of S or P,
- X4 is selected from the group consisting of E, P, K, Q or G ,
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A,
- X33 is selected from the group consisting of P, Y, H, F, L, S, G or A,
- X34 is absent or G,
- X35 is absent or T, and
- X36 is absent or Y,

Formula C1 is the following amino acid sequence as set forth in (SEQ ID NO:5): 
X0-X1-Cys()-X3-Thr-Ala-Thr-Cys()-Ala-Thr-X10-Arg-X12-Ala-X14-Phe-X16-X17-X18-X19-X20-
X21-X22-X23-X24-X25-X26-X27-X28-X29-X30-X31-Val-Gly-Ser-X35-Thr-X37 (SEQ ID NO: 5)
 wherein X represents an amino acid and;
wherein
- X0 is a basic amino acid or is absent;
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X 19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), lle(Me), Dlle(Me), Ala, DAla, Pro and DPro,or is absent;
- X25 is selected from the group consisting of Ala, DAla, Pro and DPro, or is absent;
- X26 is selected from the group consisting of Gly(Me), lle(Me), Dlle(Me), lie and Dlle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, lie, Leu, Thr, Tyr(Me), Gly(Me), Ala(Me), lle(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question;

Formula D1 is the following amino acid sequence as set forth in (SEQ ID NO:6):
Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-lle(Me)-Leu-Ser-Ser-Thr-X31-Val-Gly-Ser-X35-Thr-X37 (SEQ ID NO: 6) wherein X represents an amino acid and
wherein
- X3 is selected from the group consisting of Asn, Gly, Pro and Gln;
- X10 is selected from the group consisting of Gln, Asp and Glu;
- X14 is selected from the group consisting of Asp, His, Asn and Aad;
- X17 is selected from the group consisting of His, Asn, Gln, Glu, Thr, Val, Lys and Aad;
- X19-X20 is selected from the group consisting of Ser-Ser, Val-Val, Ser-Val and Val-Ser, or is absent;
- X31 is selected from the group consisting of Asp, Glu and Asn; X35 is selected from the group consisting of Asp, Glu, Asn, Ser, Phe, Orn, Aad, Gly and Thr; and
- X37 is selected from the group consisting of Pro, Apr and Hyp;
and wherein the compound has at least one residue selected from:
- X3 is Gln;
- X14 is His, Asn or Aad;
- X17 is Asn, Gln, Glu, Thr or Aad;
- X19-X20 is Val-Ser or Ser-Val; and
- X35 is Ser, Phe, Orn, Aad, Gly or Thr;

Formula E1 is the following amino acid sequence as set forth in (SEQ ID NO:7):
X1-X2-X3-X4-X5-X6-X7-Ala-Thr-X10-Arg-Leu-Ala-X14-Phe-Leu-X17-Arg-X19-X20-Phe-Gly(Me)-Ala-lle(Me)-X27-Ser-Ser-Thr-Glu-X32-Gly-Ser-X35-Thr-X37 (SEQ ID NO: 7) wherein X represents an amino acid and
wherein
- X1 is selected from the group consisting of Arg, Lys and Glu;
- X3 is selected from the group consisting of Gly, Gln and Pro;
- X4 is selected from the group consisting of Thr and Glu;
- X5 is selected from the group consisting of Ala and Leu;
- X6 is selected from the group consisting of Thr and Ser;
- X10 is selected from the group consisting of Glu and Gln;
- X14 is selected from the group consisting of Aad, His, Asp, Asn and Arg;
- X17 is selected from the group consisting of Gln, His and Thr;
- X19-X20 is selected from Ser-Ser, Thr-Thr, Ala-Thr, Ala-Ala, Gly-Thr, Gly-Gly and Ala-Asn or is absent;
- X27 is selected from the group consisting of Leu and Pro;
- X32 is selected from the group consisting of Val and Thr;
- X35 is selected from the group consisting of Asn and Ser;
- X37 is selected from the group consisting of Hyp and Pro; and
X2 and X7 are amino acid residues whose side chains together form a lactam bridge;
or a pharmaceutically acceptable salt or solvate thereof.

The sequence numbering corresponds to the amino acide sequence of amylin.

The sequence D1 and E1 contains a two amino acid deletion corresponding to the two residues Asn21 and Asn22 of human amylin. Thus, for ease of comparison with the amylin sequence, the Phe residue immediately C-terminal (downstream) of position X20 is designated as position 23, since it aligns with Phe23 of human amylin

In an embodiment, the short acting amylin RA chosen amongst the peptides according to formula 1 is not native human amylin.

In an embodiment, the short acting amylin RA chosen amongst the peptides according to formula 1 is not pramlintide.

Short acting amylin RA is chosen amongst the short acting amylin RAs that are disclosed in WO12168430, WO12168431, WO12168432, WO13156594, WO16034604, WO15040182, WO16146739, and corresponds to amylin RA having only a peptidic sequence.

In particular the short acting amylin RA consists of a peptidic sequence wherein the C-terminal carboxyl group is in the amide form.

In other word the short acting amylin RA does not comprise a graft comprising an acyl group wherein the acyl group may be of the RCO-type where the R group is an alkyl comprising at least at least 4 carbon atoms and may bear a carboxylic function.

In an embodiment R₁ is hydrogen.

In another embodiment R₂ is OH.

In another embodiment R₂ is NH₂.

In an embodiment R₁ is hydrogen and R₂ is OH

In an embodiment R₁ is hydrogen and R₂ is NH₂.

According to an embodiment the short acting amylin RA is chosen amongst the peptides according to Formula 1 :

**R₁-Z-R₂** Formula 1

wherein
- R₁ is hydrogen;
- R₂ is NHR₃, wherein R₃ is hydrogen or C₁₋₃-alkyl; and
- Z is an amino acid sequence according to formula A1 or B1 or C1 or D1 or E1.

According to an embodiment the short acting amylin RA is chosen amongst the peptides according to Formula 1 :

**R₁-Z-R₂** Formula 1

wherein
- R₁ is hydrogen;
- R₂ is NHR₃, wherein R₃ is hydrogen, and
- Z is an amino acid sequence according to formula A1 or B1 or C1 or D1 or E1.

The Formula 1 with Z according to Formula A1, B1, C1, D1 and E1 covers compounds disclosed in, respectively,
- WO12168430, WO12168431, WO12168432 and WO13156594,
- WO16034604
- WO15040182
- WO16146739
- WO18046719.

The applications WO12168430, WO12168431, WO12168432 and WO13156594, WO16034604, WO15040182, WO16146739, WO18046719 are incorporated by reference, in particular the peptidic sequences they disclose,

In particular the incorporation by reference of these document concerns the peptidic sequence Z as such which do not bear a graft comprising more than 6 carbon atoms.

In an embodiment the amylin RA corresponds to Formula 1 in which Z is an amino acid of sequence according to formula A1.

In an embodiment the amylin RA corresponds to Formula 1 in which R₁ is H and R₂ is NH₂, and Z is an amino acid of sequence according to formula A1.

In an embodiment the amylin RA corresponds to Formula 1 in which Z is an amino acid of sequence according to formula A1-1, said formula A1-1 being the following amino acid sequence as set forth in (SEQ ID NO:8):
Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Pro-Xaa26-Leu-Pro-Pro-Thr-Xaa31-Val-Gly-Ser-Xaa35-Thr-Xaa37) (SEQ ID NO:8) wherein Xaa represents an amino acid and
wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is Glu
- Xaa17 is independently selected from His, Arg, Lys and Val;
- Xaa18 is independently selected from Arg, Lys and His;
- Xaa21 is independently selected from Ala, Lys, Gln, Ser and Asn;
- Xaa22 is independently selected from Glu, Gln, Ser, Thr and Asn;
- Xaa26 is independently selected from Pro, Arg and Ile;
- Xaa31 is independently selected from Ser, Glu, Asp and Asn;
- Xaa35 is independently selected from His, Arg, Lys, Asp and Glu; and
- Xaa37 is independently selected from Pro and Tyr,

In an embodiment the amylin RA corresponds to Formula 1 in which R₁ is H and R₂ is NH₂, and Z is an amino acid of sequence according to formula A1-1.

In an embodiment the amylin RA corresponds to Formula 1 in which Z is an amino acid of sequence according to formula A1-2, said formula A1-2 being the following amino acid sequence as set forth in (SEQ ID NO:9):
Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Pro-Xaa26-Leu-Pro-Pro-Thr-Xaa31-Val-Gly-Ser-Xaa35-Thr-Xaa37) (SEQ ID NO:9) wherein Xaa represents an amino acid and
wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Glu and Asn;
- Xaa17 is Arg;
- Xaa18 is independently selected from Arg, Lys and His;
- Xaa21 is independently selected from Ala, Lys, Gln, Ser and Asn;
- Xaa22 is independently selected from Glu, Gln, Ser, Thr and Asn;
- Xaa26 is independently selected from Pro, Arg and Ile;
- Xaa31 is independently selected from Ser, Glu, Asp and Asn;
- Xaa35 is independently selected from His, Arg, Lys, Asp and Glu; and
- Xaa37 is independently selected from Pro and Tyr,

In an embodiment the amylin RA corresponds to Formula 1 in which R₁ is H and R₂ is NH₂, and Z is an amino acid of sequence according to formula A1-2.

In an embodiment the amylin RA corresponds to Formula 1 in which Z is an amino acid of sequence according to formula A1-3, said formula A1-3 being the following amino acid sequence as set forth in (SEQ ID NO:10): 
Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-
Xaa21-Xaa22-Phe-Gly-Pro-Xaa26-Leu-Pro-Pro-Thr-Xaa31-Val-Gly-Ser-Xaa35-Thr- (SEQ ID NO:10)
 wherein Xaa represents an amino acid and
wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Glu and Asn
- Xaa17 is independently selected from His, Arg, Lys and Val;
- Xaa18 is independently selected from Arg, Lys and His;
- Xaa21 is independently selected from Ala, Lys, Gln, Ser and Asn;
- Xaa22 is independently selected from Glu, Gln, Ser, Thr and Asn;
- Xaa26 is independently selected from Pro, Arg and Ile;
- Xaa31 is independently selected from Ser, Glu, Asp and Asn; and
- Xaa35 is independently selected from His, Arg, Lys, Asp and Glu.

In an embodiment the amylin RA corresponds to Formula 1 in which R₁ is H and R₂ is NH₂, and Z is an amino acid of sequence according to formula A1-3.

In an embodiment the amylin RA corresponds to Formula 1 in which Z is an amino acid of sequence according to formula A1-4, said formula A1-4 being the following amino acid sequence as set forth in (SEQ ID NO:11):
Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Xaa25-Xaa26-Xaa27-Xaa28-Xaa29-Thr-Xaa31-Val-Gly- Xaa34-Xaa35-Thr-Xaa37 (SEQ ID NO:11) wherein Xaa represents an amino acid and
wherein
- Xaa1 is independently selected from Ala, Cys, Glu, Gly, His, Arg, Ser and Lys;
- Xaa3 is Asn;
- Xaa14 is independently selected from Asp, Glu, His, Asn, Arg, Gly, Ala, Ser, Lys, Thr and Cys;
- Xaa17 is independently selected from Arg and Val;
- Xaa18 is His;
- Xaa21 is Pro;
- Xaa22 is Asn;
- Xaa25 is independently selected from Pro and Ala
- Xaa26 is independently selected from Pro and Ile;
- Xaa27 is independently selected from Pro and Leu;
- Xaa28 is independently selected from Pro and Ser;
- Xaa29 is independently selected from Pro and Ser;
- Xaa31 is independently selected from Pro and Asn;
- Xaa34 is independently selected from Pro, His, Lys, Arg and Ser;
- Xaa35 is independently selected from Asp, Arg, Glu, Lys, His and Asn; and
- Xaa37 is independently selected from Pro and Tyr.

In an embodiment the amylin RA corresponds to Formula 1 in which R₁ is H and R₂ is NH₂, and Z is an amino acid of sequence according to formula A1-4.

In an embodiment the amylin RA has a sequence corresponding to [Glu14,His17,His35]-pramlintide (Cpd 1).

In an embodiment the amylin RA has a sequence corresponding to [Glu14,Arg17,His35]-pramlintide (Cpd 2).

In an embodiment the amylin RA has a sequence corresponding to [Glu14,His17,Pro37]-pramlintide (Cpd 3).

In an embodiment the amylin RA has a sequence corresponding to [Arg1,Glu14,His17,Pro37]-pramlintide (Cpd 4); disclosed as Example 96 in WO 2012/168430 A2.

In an embodiment the amylin RA has a sequence corresponding to [Glu14,Arg17,Pro37]-pramlintide (Cpd 5).

In an embodiment the amylin RA is Cpd 5 and has the following sequence :

Lys-Cys-Asn-Thr-Ala-Thr-Cys-Ala-Thr-Gln-Arg-Leu-Ala-Glu-Phe-Leu-Arg-His-Ser-Ser-Asn-Asn-
Phe-Gly-Pro-Ile-Leu-Pro-Pro-Thr-Asn-Val-Gly-Ser-Asn-Thr-Pro-NH2. (SEQ ID NO: 12) .

In an embodiment the amylin RA corresponds to Formula 1 in which Z is an amino acid of sequence according to formula B1.

In an embodiment the amylin RA corresponds to Formula 1 in which R₁ is H and R₂ is NH₂, and Z is an amino acid of sequence according to formula B1.

In an embodiment the amylin RA corresponds to Formula 1 in which Z is an amino acid of sequence according to formula B1-1, said formula B1-1 being the following amino acid sequence as set forth in (SEQ ID NO:13): 
X(-1)-X1-X2-X3-X4-X5-X6-X7-X8-X9-X10-X11-X12-X13-X14-X15-X16-X17-X18-X19-X20-X21-
X22-X23-X24-X25-X26-X27-X28-X29-X30-X31-X32-X33-X34 (SEQ IDNO: 13),
 wherein X represents an amino acid and
wherein
- X(-1) is absent or E,
- X1 is absent or selected from the group consisting of E or A,
- X2 is selected from the group consisting of L, A or P,
- X3 is selected from the group consisting of S or P
- X4 is selected from the group consisting of E, P, K, Q or G
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A and
- X33 is selected from the group consisting of P, Y, H, F, L, S, G or A and
- X34 is absent or G.

In an embodiment the amylin RA corresponds to Formula 1 in which R₁ is H and R₂ is NH₂, and Z is an amino acid of sequence according to formula B1-1.

In an embodiment the amylin RA corresponds to Formula 1 in which Z is an amino acid of sequence according to formula B1-2, said formula B1-2 being the following amino acid sequence as set forth in (SEQ ID NO:14):
X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36 (SEQ ID NO: 14) wherein X represents an amino acid and
wherein
- X1 is absent or selected from the group consisting of E or A,
- X2 is selected from the group consisting of L, A or P,
- X3 is selected from the group consisting of S or P
- X4 is selected from the group consisting of E, P, K, Q or G
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I, X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A,
- X33 is selected from the group consisting of P, Y, H, F, L, S, G or A,
- X34 is absent or G,
- X35 is absent or T and
- X36 is absent or Y.

In an embodiment the amylin RA corresponds to Formula 1 in which R₁ is H and R₂ is NH₂, and Z is an amino acid of sequence according to formula B1-2.

In an embodiment the amylin RA corresponds to Formula 1 in which Z is an amino acid of sequence according to formula B1-3, said formula B1-3 being the following amino acid sequence as set forth in (SEQ ID NO:15):
X1-X2-X3-X4-X5-X6-T-X8-X9-X10-G-X12-L-X14-X15-X16-X17-X18-X19-X20-X21-T-X23-P-X25-T-X27-X28-G-X30-X31-X32-X33 (SEQ ID NO: 15), wherein X represents an amino acid and
wherein
- X1 is absent or selected from the group consisting of E or A,
- X2 is selected from the group consisting of L, A or P,
- X3 is selected from the group consisting of S or P,
- X4 is selected from the group consisting of E, P, K, Q or G,
- X5 is selected from the group consisting of L, V, or I,
- X6 is selected from the group consisting of S, T or H,
- X8 is selected from the group consisting of L or A,
- X9 is selected from the group consisting of A, V, I, S or T,
- X10 is selected from the group consisting of L, A, I, H or V,
- X12 is selected from the group consisting of R, H or K,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X23 is selected from the group consisting of T, Y or L,
- X25 is selected from the group consisting of R, P, H or K,
- X27 is selected from the group consisting of E, Q, G or K,
- X28 is selected from the group consisting of T or P,
- X30 is selected from the group consisting of P, S or T,
- X31 is selected from the group consisting of E, Q, G, A, P or K,
- X32 is selected from the group consisting of T, S, H, P or A and
- X33 is selected from the group consisting of P, Y, H, F, L, S, G or A.

In an embodiment the amylin RA corresponds to Formula 1 in which R₁ is H and R₂ is NH2, and Z is an amino acid of sequence according to formula B1-3.

In an embodiment the amylin RA is mimylin. This amylin RA has the sequence EASELSTAALGRLSAELHELATLPRTETGPESP-NH₂ SEQ ID NO: 16 (Cpd 6).

In an embodiment the amylin RA is a peptide comprising at least 66% sequence identity to EASELSTAALGRLSAELHELATLPRTETGPESP SEQ ID NO: 17

In an embodiment the amylin RA is a peptide comprising at least 66% sequence identity to EASELSTAALGRLSAELHELATLPRTETGPESP-NH₂ SEQ ID NO: 18

In an embodiment the amylin RA corresponds to Formula 1 in which Z is an amino acid of sequence according to formula C1.

In an embodiment the amylin RA corresponds to Formula 1 in which R₁ is H and R₂ is NH₂, and Z is an amino acid of sequence according to formula C1.

In an embodiment the amylin RA corresponds to Formula 1 in which Z is an amino acid of sequence according to formula C1-1, said formula C1-1 being the following amino acid sequence as set forth in (SEQ ID NO:19): 
X0-X1-Cys()-X3-Thr-Ala-Thr-Cys()-Ala-Thr-X10-Arg-X12-Ala-X14-Phe-X16-X17-X18-X19- X20-
X21-X22-X23-X24-X25-X26-X27-X28-X29-X30-X31-Val-Gly-Ser-X35-Thr-X37 (SEQ ID NO: 19)
 wherein X represents an amino acid and
wherein
- X0 is a basic amino acid or is absent;
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), lle(Me), Dlle(Me), Ala, DAla, Pro and DPro,or is absent;
- X25 is selected from the group consisting of Ala, DAla, Pro and DPro, or is absent;
- X26 is selected from the group consisting of Gly(Me), lle(Me), Dlle(Me), lie and Dlle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, lie, Leu, Thr, Tyr(Me), Gly(Me), Ala(Me), lle(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question.

In an embodiment the amylin RA corresponds to Formula 1 in which R₁ is H and R₂ is NH₂, and Z is an amino acid of sequence according to formula C1-1.

In an embodiment the amylin RA corresponds to Formula 1 in which Z is an amino acid of sequence according to formula C1-2 said formula C1-2 being the following amino acid sequence as set forth in (SEQ ID NO:20) 
X1-Cys()-X3-Thr-Ala-Thr-Cys()-Ala-Thr-X10-Arg-X12-Ala-X14-Phe-X16-X17-X18-X19-X20- X21
-X22-X23-X24-X25-X26-X27-X28-X29-X30-X31-Val-Gly-Ser-X35-Thr-X37 (SEQ ID NO: 20)
 wherein X represents an amino acid and;
wherein
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), lle(Me), Dlle(Me), Ala, DAla, Pro and DPro,or is absent;
- X25 is selected from the group consisting of Ala, DAla, Pro and DPro, or is absent;
- X26 is selected from the group consisting of Gly(Me), lle(Me), Dlle(Me), lie and Dlle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent; X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, lie, Leu, Thr, Tyr(Me), Gly(Me), Ala(Me), lle(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question.

In an embodiment the amylin RA corresponds to Formula 1 in which R₁ is H and R₂ is NH₂, and Z is an amino acid of sequence according to formula C1-2.

In an embodiment in Formula C1, C1-1 and C1-2 at least one peptide bond in the peptide backbone of the amino acid sequence of Z is N-methylated; and that the side-chain of at least one amino acid residue that is other than the amino acid residue bearing the N-methyl group of the N-methylated peptide bond on its N-alpha atom and that is located at a sequence position from X19 to X37 of Z is different from the side-chain of the amino acid residue at the corresponding position in human amylin ; or a pharmaceutically acceptable salt or solvate thereof.

In an embodiment the amylin RA corresponds to Formula 1 in which Z is an amino acid of sequence according to formula C1-3 said formula C1-3 being the following amino acid sequence as set forth in (SEQ ID NO:21): 
X1-Cys()-X3-Thr-Ala-Thr-Cys()-Ala-Thr-X10-Arg-Leu-Ala-X14-Phe-X16-X17-X18-X19-X20-X21-
X22-X23-X24-X25-X26-X27-X28-X29-X30-X31-Val-Gly-Ser-X35-Thr-X37; (SEQ ID NO: 21)
 wherein X represents an amino acid and
wherein
- X1 is selected from the group consisting of Trp, Lys and Arg;
- X3 is selected from the group consisting of Gly, Asn, Glu and Pro;
- X10 is selected from the group consisting of Gln, Asp and Glu;
- X14 is selected from the group consisting of Asp and Glu;
- X16 is Leu or is absent;
- X17 is His or is absent;
- X18 is selected from the group consisting of His and Arg, or is absent;
- X19 is selected from the group consisting of Ser and D-Ser, or is absent;
- X20 is Ser or is absent;
- X21 is selected from the group consisting of Asn and Ser, or is absent;
- X22 is selected from the group consisting of Asn and Ser, or is absent;
- X23 is selected from the group consisting of Phe and D-Phe, or is absent;
- X24 is Gly(Me) or is absent;
- X25 is Ala or is absent;
- X26 is lle(Me) or is absent;
- X27 is Leu or is absent;
- X28 is selected from the group consisting of Ser, Ser(Me), D-Ser and D-Ser(Me), or is absent;
- X29 is selected from the group consisting of Ser and Ser(Me) or is absent;
- X30 is Thr or is absent;
- X31 is selected from the group consisting of Asp, Glu and Asn;
- X35 is selected from the group consisting of Asp, Glu and Asn; and
- X37 is selected from the group consisting of Tyr, Tyr(Me), Pro, Hyp, Apr, Aze, Pip, Php and Bep;
wherein the parentheses () associated with the two cysteine (C, Cys) residues at sequence positions 2 and 7 indicate the presence of an intramolecular disulfide bridge between the two Cys residues in question and whereinno more than three positions may be absent.

In an embodiment the amylin RA corresponds to Formula 1 in which R₁ is H and R₂ is NH₂, and Z is an amino acid of sequence according to formula C1-3.

In an embodiment in Formula C1-3, no more than three positions may be absent, with the proviso that:
(i) if two positions are absent, they are adjacent to one another or one of the absent positions is X25; and
(ii) if three positions are absent, two are adjacent to one another and the other is X25;
or a pharmaceutically acceptable salt or solvate thereof.

In an embodiment the amylin RA corresponds to Formula 1 in which Z is an amino acid of sequence according to formula C1-4 said formula C1-4 being the following amino acid sequence as set forth in (SEQ ID NO:22):
X1-Cys()-X3-Thr-Ala-Thr-Cys()-Ala-Thr-X10-Arg-Leu-Ala-Glu-Phe-Leu-His-X18-Ser-Ser- X21-X22-X23-Gly(Me)-Ala-lle(Me)-Leu-X28-Ser-Thr-X31-Val-Gly-Ser-X35-Thr-X37 (); (SEQ ID NO: 22) wherein X represents an amino acid and
wherein
- X1 is selected from the group consisting of Trp, Lys and Arg;
- X3 is selected from the group consisting of Gly, Asn and Pro;
- X10 is selected from the group consisting of Gln, Asp and Glu;
- X18 is selected from the group consisting of His and Arg;
- X21 is selected from the group consisting of Asn and Ser, or is absent;
- X22 is selected from the group consisting of Asn and Ser, or is absent;
- X23 is selected from the group consisting of Phe and D-Phe;
- X28 is selected from the group consisting of Ser and D-Ser;
- X31 is selected from the group consisting of Asp, Glu and Asn;
- X35 is selected from the group consisting of Asp, Glu and Asn; and
- X37 is selected from the group consisting of Pro and Hyp.

In an embodiment the amylin RA corresponds to Formula 1 in which R₁ is H and R₂ is NH₂, and Z is an amino acid of sequence according to formula C1-4.

In any of the above formulae C1, C1-1 to C1-4 (except where stated otherwise), the following residues or combinations of residues may be employed:
- X16 is Leu and/or X30 is Thr, e.g. X16 is Leu and X30 is Gly.
- X3 is selected from Asn, Gly and Pro, e.g. X3 is Asn.
- X10 is Gln.
- X14 is Glu.
- X23 is D-Phe and/or X28 is D-Ser. For example, X23 is D-Phe and X28 is D-Ser.
- X21 and/or X22 are absent. For example, X21 and X22 are absent.
- X21 and/or X22 are Asn. For example, X21 and X22 are Asn.
- X21 and/or X22 are Ser. For example, X21 and X22 are Ser.
- X19 and/or X20 are absent. For example. X19 and X20 are absent. (Not applicable for formula C1-4)
- X19 and/or X20 are Ser. For example, X19 and X20 are Ser.
- X25 is absent. (Not applicable for formula C1-4).
- X31 and/or X35 are Glu. For example, X31 and X35 are Glu.
- X31 and/or X35 are Asp. For example, X31 and X35 are Asp.
- X31 and/or X35 are Asn. For example, X31 and X35 are Asn.

In certain embodiments of a compound or pharmaceutically acceptable salt or solvate according to formula C1 and C1-1 to C1-4:
- X0 is selected from the group consisting of Lys, His and Arg, or is absent;
- X1 is selected from the group consisting of Trp, Lys and Arg;
- X3 is selected from the group consisting of Gly, Asn, Glu and Pro;
- X10 is selected from the group consisting of Asp and Glu;
- X12 is Leu;
- X14 is Glu;
- X16 is Leu;
- X17 is His; X18 is Arg;
- X19 is Ser;
- X20 is Ser;
- X21 is selected from the group consisting of Asn and Ser, or is absent;
- X22 is selected from the group consisting of Asn and Ser, or is absent;
- X23 is Phe;
- X24 is Gly(Me);
- X25 is Ala, or is absent;
- X26 is lle(Me);
- X27 is Leu;
- X28 is Ser;
- X29 is Ser;
- X30 is Thr;
- X31 is selected from the group consisting of Asp, Glu and Asn ;
- X35 is selected from the group consisting of Asp, Glu and Asn ;
- X37 is selected from the group consisting of Tyr, Pro, Hyp and Apr.

In an embodiment the amylin RA corresponds to Formula 1 in which Z is an amino acid of sequence according to formula C1-5, said formula C1-5 being the following amino acid sequence as set forth in (SEQ ID NO:23): 
X1-Cys()-Asn-Thr-Ala-Thr-Cys()-Ala-Thr-Gln-Arg-Leu-Ala-X14-Phe-X16-X17-X18-X19-X20-
X21-X22-X23-X24-X25-X26-X27-X28-X29-X30-X31-Val-Gly-Ser-X35-Thr-X37 (SEQ ID NO: 23)
 wherein X represents an amino acid and
wherein
- X1 is a basic amino acid;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Pro, DPro, Ala, DAla, Ala(Me), DAla(Me), Gly(Me), lle(Me) and Dlle(Me), or is absent
- X25 is selected from the group consisting of Ala, DAla, Pro and DPro, or is absent; X26 is selected from the group consisting of Gly(Me), lle(Me), Dlle(Me) and Dlle, or is absent;
- X27 is selected from the group consisting of Leu and DLeu, or is absent;
- X28 is selected from the group consisting of Pro, DPro, Ser, DSer, Ser(Me) and DSer(Me), or is absent;
- X29 is selected from the group consisting of Pro, DPro, Ser, DSer, Ser(Me) and DSer(Me), or is absent;
- X30 is selected from the group consisting of Thr and DThr, or is absent;
- X31 is selected from the group consisting of Asn, DAsn, Glu, DG1Iu, Asp and DAsp, or is absent;
- X35 is selected from the group consisting of His, DHis, Asn, DAsn, Glu, DGlu, Asp and DAsp, or is absent; and
- X37 is selected from the group consisting of Tyr, DTyr, Pro and DPro, or is absent.

In an embodiment the amylin RA corresponds to Formula 1 in which R₁ is H and R₂ is NH₂, and Z is an amino acid of sequence according to formula C1-5.

In an embodiment, in Formula C1-5 the peptide bond formed by at least one amino acid residue in sequence positions X19 to X37 of the amino acid sequence of Z is *N*-methylated; and that at least one other of the amino acid residues in sequence positions X19 to X37 of Z is different from the amino acid residue at the corresponding position in the sequence of human amylin; and wherein at most 5 amino acid residues at positions X17 to X31 , X35 and X37 are absent; or a pharmaceutically acceptable salt or solvate thereof.

In some embodiments in Formula C1-5 among this latter group of embodiments, X1 is a basic amino acid selected from the group consisting of Lys, DLys, Orn, DOrn, Dap, DDap, Dab, DDab, His, DHis, Arg and DArg.

In some embodiments in Formula C1-5 among this latter group of embodiments, X24 is Gly(Me).

In some embodiments in Formula C1-5 among this latter group of embodiments, X26 is lle(Me).

In some embodiments in Formula C1-5 among this latter group of embodiments, X24 is Gly(Me) and X26 is lle(Me).

In some embodiments in Formula C1-5 among this latter group of embodiments, X25 is Ala or is absent.

In some embodiments in Formula C1-5 among this latter group of embodiments, X14 is Glu.

In an embodiment in Formula C1 and C1-5 the peptide bond formed by at least one amino acid residue in sequence positions X19 to X37 of the amino acid sequence of Z is N-methylated; and that at least one other of the amino acid residues in sequence positions X19 to X37 of Z is different from the amino acid residue at the corresponding position in the sequence of human amylin; and wherein at most 5 amino acid residues at positions X17 to X31 , X35 and X37 are absent.

In an embodiment in Formula C1 and C1-1 to C1-5 there isno more than 5 positions which are absent.

In an embodiment in Formula C1 and C1-1 to C1-5 there isno more than 4 positions which are absent.

In an embodiment in Formula C1 and C1-1 to C1-5 there is no more than 3 positions which are absent.

In an embodiment, the amylin RA has the following sequence RC()NTATC()ATQRLAEFLHRSSF-Gly(Me)-A-lle(Me)-LSSTEVGSETP-NH₂ (SEQ ID No: 24) (Cpd 7).

This sequence is the peptidic sequence of Compound 82 from WO2015040182.

In an embodiment, the amylin RA has the following sequence RC()NTATC()ATQRLAEFLHRSSNNF-Gly(Me)-A-lle(Me)-LSSTNVGSNTP-NH₂ (SEQ ID NO: 25) (Cpd 8).

This sequence is the peptidic sequence of Compound 18 from WO2015040182.

In an embodiment the amylin RA corresponds to Formula 1 in which Z is an amino acid of sequence according to formula D1.

In an embodiment the amylin RA corresponds to Formula 1 in which R₁ is H and R₂ is NH₂, and Z is an amino acid of sequence according to formula D1.

In an embodiment the amylin RA corresponds to Formula 1 in which Z is an amino acid of sequence according to formula D1-1, said formula D1-1 being the following amino acid sequence as set forth in (SEQ ID NO:26):
Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-lle(Me)-Leu-Ser-Ser-Thr-X31-Val-Gly-Ser-X35-Thr-X37 (SEQ ID NO: 26) wherein X represents an amino acid and
wherein
- X3 is selected from the group consisting of Asn, Gly, Pro and Gln;
- X10 is selected from the group consisting of Gln, Asp and Glu;
- X14 is selected from the group consisting of Asp, His, Asn and Aad;
- X17 is selected from the group consisting of His, Asn, Gln, Glu, Thr, Val, Lys and Aad;
- X19-X20 is selected from the group consisting of Ser-Ser, Val-Val, Ser-Val and Val-Ser, or is absent;
- X31 is selected from the group consisting of Asp, Glu and Asn;
- X35 is selected from the group consisting of Asp, Glu, Asn, Ser, Phe, Orn, Aad, Gly and Thr; and
- X37 is selected from the group consisting of Pro, Apr and Hyp;
or a pharmaceutically acceptable salt or solvate thereof.

In an embodiment the amylin RA corresponds to Formula 1 in which R₁ is H and R₂ is NH₂, and Z is an amino acid of sequence according to formula D1-1.

According to an embodiment the peptide according to Formula D1-1 has at least one residue selected from:
- X3 is Gln;
- X4 is His, Asn or Aad;
- X17 is Asn, Gln, Glu, Thr or Aad;
- X19-X20 is Val-Ser or Ser-Val; and
- X35 is Ser, Phe, Orn, Aad, Gly or Thr;
or a pharmaceutically acceptable salt or solvate thereof.

In some embodiments according to formula D1-1 it may be desirable that: X31 is Glu; X19-X20 is Ser-Ser or is absent; and/or X37 is Hyp or Pro.

It may be desirable that the amylin analogue contains at least one of His14, Asn14, Aad14, Gln17 and Thr17.

If X14 is Asp, then it may be desirable that X17 is Asn, Gln, Glu, Thr or Aad.

X17 is Gln may be particularly preferred. In some circumstances, it may be desirable that X35 is not a hydrophobic residue, e.g. Phe. Such residues may increase tendency towards fibrillation in some formulations.

In an embodiment the amylin RA corresponds to Formula 1 in which Z is an amino acid of sequence according to formula D1-2 said formula D1-2 being the following amino acid sequence as set forth in (SEQ ID NO:27):
Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-lle(Me)-Leu-Ser-Ser-Thr-Glu-Val-Gly-Ser-X35-Thr-X37 ( (SEQ ID NO: 27) wherein X represents an amino acid and
wherein
- X3 is selected from the group consisting of Asn, Gly and Gln;
- X10 is selected from the group consisting of Gln, Asp and Glu;
- X14 is selected from the group consisting of Asp, His and Aad;
- X17 is selected from the group consisting of His, Asn, Gln, Glu, Lys and Aad;
- X19-X20 is Ser-Ser or is absent;
- X35 is selected from the group consisting of Asp, Glu, Asn, Ser, Orn, Aad, Gly and Thr; and
- X37 is selected from the group consisting of Pro and Hyp;
and wherein the compound has at least one residue selected from:
- X3 is Gln;
- X14 is His or Aad;
- X17 is Asn, Gln, Glu or Aad; and
- X35 is Ser, Phe, Orn, Aad, Gly or Thr.

In an embodiment the amylin RA corresponds to Formula 1 in which R₁ is H and R₂ is NH₂, and Z is an amino acid of sequence according to formula D1-2.

In some embodiments according to formula D1-2, X17 may be selected from His and Gln.

In an embodiment the amylin RA corresponds to Formula 1 in which Z is an amino acid of sequence according to formula D1-3, said formula D1-3 being the following amino acid sequence as set forth in (SEQ ID NO:28): 
Arg-Cys-X3-Thr-Ala-Thr-Cys-Ala-Thr-X10-Arg-Leu-Ala-X14-Phe-Leu-X17-Arg-X19-X20-        Phe-
Gly(Me)-Ala-Ile(Me)-Leu-Ser-Ser-Thr-Glu-Val-Gly-Ser-X35-Thr-X37; (SEQ ID NO: 28)
 wherein
- X3 is selected from the group consisting of Asn, Gly and Gln;
- X10 is selected from the group consisting of Gln, Asp and Glu;
- X14 is selected from the group consisting of Asp, His and Aad;
- X17 is selected from the group consisting of His and Gln;
- X19-X20 is Ser-Ser or is absent;
- X35 is selected from the group consisting of Asp, Glu, Asn, Aad and Gly; and
- X37 is selected from the group consisting of Pro and Hyp;
and wherein the compound has at least one residue selected from:
- X3 is Gln;
- X14 is His or Aad;
- X17 is Gln; and
- X35 is Aad.

In an embodiment the amylin RA corresponds to Formula 1 in which R₁ is H and R₂ is NH₂, and Z is an amino acid of sequence according to formula D1-3.

In particular the short-acting amylin RA according to Formula D1-1 to D1-3 corresponds to the amino acid sequences disclosed in WO2016146739, in particular from pages 9-12, which are incorporated by reference.

In an embodiment, the short acting amylin RA is a peptide having a sequence corresponding to the peptidic sequence of Compound 6, 7, 9, 11, 13, 15, 28, 34, 36, 49, 55, 57, 59, 71 or 79 disclosed in WO2016146739, which is incorporated by reference.

In an embodiment this short acting amylin RA corresponds to Formula 1 with R₁ being H and R₂ being NH₂.

In an embodiment the short acting amylin RA has the following sequence, R-C()-GTAT-C()-ATERLAHFLQRSSF-Gly(Me)-A-lle(Me)-LSSTEVGSNT-Hyp-NH₂ (SEQ ID NO: 29) (Cpd 9).

This sequence is the peptidic sequence of Compound 7 of WO2016146739.

In an embodiment the amylin RA corresponds to Formula 1 in which Z is an amino acid of sequence according to formula E1.

In an embodiment of Formula E1, one of the residues at positions X2 and X7 is a residue with a side chain comprising a carboxylic acid group and the other is a residue with a side chain comprising an amine group, wherein a lactam (cyclic amide) is formed between the carboxylic acid and amine groups. The amine may be a primary or secondary amine, but is typically a primary amine. Suitable amino acids whose side chains can participate in a lactam bridge include Asp, Glu and Aad (having side chains comprising carboxylic acid groups) and Dap, Dab, Orn, Lys and hLys (having side chains comprising amine groups). Any of the amino acids selected from Asp, Glu and Aad may in principle form a lactam bridge with any of the amino acids selected from the group consisting of Dap, Dab, Orn, Lys and hLys.

In an embodiment in Formula E1, X2 is selected from Asp, Glu and Aad and X7 is selected from Dap, Dab, Orn, Lys and hLys.

In an embodiment the amylin RA corresponds to Formula 1 in which R₁ is H and R₂ is NH₂, and Z is an amino acid of sequence according to formula E1.

In an embodiment the amylin RA corresponds to Formula 1 in which Z is an amino acid of sequence according to formula E1-1 said formula E1-1 being the following amino acid sequence as set forth in (SEQ ID NO:30):
X1-X2-X3-X4-X5-X6-X7-Ala-Thr-X10-Arg-Leu-Ala-X14-Phe-Leu-X17-Arg-X19-X20-Phe-Gly(Me)-Ala-lle(Me)-X27-Ser-Ser-Thr-Glu-X32-Gly-Ser-X35-Thr-X37 (SEQ ID NO: 30) wherein X represents an amino acid and
wherein
- X1 is selected from the group consisting of Arg, Lys and Glu; X3 is selected from the group consisting of Gly, Gln and Pro;
- X4 is selected from the group consisting of Thr and Glu;
- X5 is selected from the group consisting of Ala and Leu;
- X6 is selected from the group consisting of Thr and Ser; X10 is selected from the group consisting of Glu and Gln;
- X14 is selected from the group consisting of Aad, His, Asp, Asn and Arg;
- X17 is selected from the group consisting of Gln, His and Thr;
- X19-X20 is selected from Ser-Ser, Thr-Thr, Ala-Thr, Ala-Ala, Gly-Thr, Gly-Gly and Ala-Asn or is absent;
- X27 is selected from the group consisting of Leu and Pro;
- X32 is selected from the group consisting of Val and Thr;
- X35 is selected from the group consisting of Asn and Ser;
- X37 is selected from the group consisting of Hyp and Pro;
and
- X2 and X7 are amino acid residues whose side chains together form a lactam bridge;
or a pharmaceutically acceptable salt or solvate thereof.

In an embodiment the amylin RA corresponds to Formula 1 in which R₁ is H and R₂ is NH₂, and Z is an amino acid of sequence according to formula E1-1.

In some embodiments, X1 is selected from Arg and Lys.

In some embodiments, X3 is Gly, X4 is Thr, X5 is Ala and/or X6 is Thr, e.g. X3 is Gly, X4 is Thr, X5 is Ala and X6 is Thr.

In some embodiments, X14 is selected from His, Asp and Aad.

In some embodiments, X17 is Gin.

In some embodiments, X19-X20 is selected from Ser-Ser and Thr-Thr, or is absent, e.g. Ser-Ser.

In some embodiments, X32 is Val, X35 is Asn and/or X37 is Hyp.

In an embodiment the amylin RA corresponds to Formula 1 in which Z is an amino acid of sequence according to formula E1-2 said formula E1-2 being the following amino acid sequence as set forth in (SEQ ID NO: 31):
X1-X2-Gly-Thr-Ala-Thr-X7-Ala-Thr-X10-Arg-Leu-Ala-X14-Phe-Leu-Gln-Arg-X19-X20-Phe-Gly(Me)-Ala-lle(Me)-X27-Ser-Ser-Thr-Glu-Val-Gly-Ser-Asn-Thr-Hyp (SEQ ID NO: 31) wherein X represents an amino acid and
wherein
- X1 is selected from the group consisting of Arg and Lys;
- X10 is selected from the group consisting of Glu and Gln;
- X14 is selected from the group consisting of Aad, Asp and His;
- X19-X20 is selected from Ser-Ser and Thr-Thr or is absent;
- X27 is selected from the group consisting of Leu and Pro; and
- X2 and X7 are amino acid residues whose side chains together form a lactam bridge.

In an embodiment the amylin RA corresponds to Formula 1 in which R₁ is H and R₂ is NH₂, and Z is an amino acid of sequence according to formula E1-2.

In some embodiments, X14 is Aad, X19-X20 is Ser-Ser and X27 is Leu.

In particular the specific pairing of short-acting amylin RA according to Formula E1-1 to E1-2 are disclosed in WO2018046719, in particular from pages 8-9, which are incorporated by reference

In particular the short-acting amylin RA according to Formula E1-1 to E1-2 corresponds to the amino acid sequences disclosed in WO2018046719, in particular from pages 9-13, which are incorporated by reference.

In an embodiment the short acting amylin RA has the following sequence, RD()GTATK()ATQRLADFLQRSSF-Gly(Me)-A-lle(Me)-LSSTE-VGSNT-Hyp-NH₂ (SEQ ID NO: 32) (Cpd 10) corresponding to the peptidic sequence of Compd. 41 of WO18046719.

In an embodiment the short acting amylin RA has the following sequence, RD()GTATK()ATERLA-Aad-FLQRSSF-Gly(Me)-A-lle(Me)-LSSTEVGSNT-Hyp-NH₂ (SEQ ID NO: 33) (Cpd 11) corresponding to the peptidic sequence of Compd. 35 of WO18046719.

In an embodiment the short acting amylin RA has the following sequence, RD()GTATK()ATERLA-Aad-FLQRTTF-Gly(Me)-A-lle(Me)-LSSTEVGSNT-Hyp-NH₂ (SEQ ID NO: 34) (Cpd 12) corresponding to the peptidic sequence of Compd. 38 of WO18046719.

In an embodiment the short acting amylin RA has the following sequence, RD()GTATK()ATERLAHFLQRSSF-Gly(Me)-A-lle(Me)-LSSTE-VGSNT-Hyp-NH₂ (SEQ ID NO: 35) (Cpd 13) corresponding to the peptidic sequence of Compd. 18 of WO18046719.

In an embodiment the short acting amylin RA has the following sequence, KD()GTATK()ATQRLA-Aad-FLQRSSF-Gly(Me)-Alle(Me)-LSSTEVGSNTHyp-_{NH2} (SEQ ID NO: 36) (Cpd 14) corresponding to the peptidic sequence of Compd. 39 of WO18046719.

In an embodiment, the short acting amylin RA corresponds to Formula I with R₁ = H, R₂ = NH₂ and Z is the peptide sequence of ZP8396.

Throughout the present specification "natural amino acid" is an amino acid (with the usual three letter codes & one letter codes in parenthesis) selected from the group consisting of: Glycine (Gly & G), proline (Pro & P), alanine (Ala & A), valine (Val & V), leucine (Leu & L), isoleucine (Ile & I), methionine (Met & M), cysteine (Cys & C), phenylalanine (Phe & F), tyrosine (Tyr & Y ), tryptophan (Trp & W), histidine (His & H), lysine (Lys & K), arginine (Arg & R), glutamine (Gln & Q), asparagine (Asn & N), glutamic acid (Glu & E), aspartic acid (Asp & D), serine (Ser & S) and threonine (Thr & T). If, due to typing errors, there are deviations from the commonly used codes, the commonly used codes apply. The amino acids present in the polypeptides of the present invention are, preferably, amino acids which can be coded for by a nucleic acid.

Less common or non-naturally occurring amino acids may be used, unless they are referred to by their full name (e.g. sarcosine, ornithine, etc.), the three- or four-character codes may be employed for residues thereof, including Orn (ornithine, i.e. 2,5-diaminopentanoic acid), Aib (a-aminoisobutyric acid), Dab (2,4-diaminobutanoic acid), Dap (2,3-diaminopropanoic acid), Har (homoarginine), γ-Glu (γ-glutamic acid), Gaba (γ-aminobutanoic acid), β-Ala (i.e. 3-aminopropanoic acid) and 8Ado (8-amino-3,6- dioxaoctanoic acid).

The term "basic amino acid" as employed in the context of the present invention refers to an amino acid containing one or more additional basic groups. Examples thereof include Lys, DLys, Orn, DOrn, Dap, DDap, Dab, DDab, His, DHis, Arg and DArg. Further examples thereof include guanidino derivatives of such basic amino acids, e.g. 2-amino-4-guanidino-1-butyric acid (Guanidino analog of L-Dab) or 2-amino-3-guanidino-1-propionic acid (Guanidino analog of L-Dap).

Among other than natural amino acids may be cited:
- Ala(Me): N-methylalanine,
- Gly(Me): N-methylglycine, also called sarcosine (Sar),
- lle(Me): N-methylisoleucine,
- Ser(Me): N-methylserine,
- Tyr(Me): N-methyltyrosine,
- Apr: 4-aminoproline, e.g. (2S,4R)-4-aminoproline, also called (4R)-4-amino-L-proline,
- Aze: azetidine-2-carboxylic acid, e.g. (2S)-azetidine-2-carboxylic acid,
- Bep: 4-benzylproline, e.g. (2S,4R)-4-benzylproline, also called (4R)-4-benzyl-L-proline,
- Hyp: 4-hydroxyproline, e.g. (2S,4R)-4-hydroxyproline, also called (4R)-4-hydroxy-L-proline,
- Php: 4-phenylproline, e.g. (2S,4S)-4-phenylproline, also called (4S)-4-phenyl-L-proline
- Pip: (2S)-piperidine-2-carboxylic acid, also called (S)-pipecolic acid,
- Aad: 2-aminoadipic acid, e.g. (2S)-2-aminoadipic acid [also (2S)-2-aminohexanedioic,
- hLys: 2-amino-7-amino-heptanoic acid, also known as homo-lysine, e.g. (2S)-2-amino-7-amino-heptanoic acid.

Unless otherwise indicated, reference is made to the L-isomeric forms of the amino acids in question. Where appropriate, the D-isomeric form of an amino acid is indicated in the conventional manner by the prefix "D" before the conventional three-letter code (e.g. DAsp, DPhe). In some case the d isoform of Glu is written "e" in the sequence.

If the amylin RA contains either more than 37 amino acid residues or less than 37 amino acid residues then the skilled person can still align that sequence with the sequence of pramlintide (SEQ ID NO: 2) to determine the placement number of the corresponding, respective amino acid residue. A suitable alignment program is "needle", which is a Needleman-Wunsch alignment. The algorithm for this alignment program is described in Needleman, S.B. and Wunsch, CD., (1970), Journal of Molecular Biology, 48: 443-453.

In the numbering sequence of SEQ ID NO: X, and according to established practice in the art, the amino acid residue at the N-terminal is assigned no. 1 and subsequent amino acid residues are numbered consecutively, ending at the C-terminal.

According to an embodiment, the short acting amylin RA has an EC50 in a human amylin receptor potency assay, such as that disclosed in WO2012168430, of about 1800pM or less.

According to an embodiment, the short acting amylin RA has an EC50 in a human calcitonin receptor potency assay, such as that disclosed in WO2012168430, of about 1800pM or less.

According to an embodiment, the short acting amylin RA has a solubility at pH 4 of about 100 µM or more in a solubility assay, such as that disclosed in WO2012168430.

According to an embodiment, the short acting amylin RA has a solubility at pH 7 of about 100 µM or more in a solubility assay, such as that disclosed in WO2012168430.

According to an embodiment, the short acting amylin RA has a physical stability of about 25 hours or greater in a fibrillogenesis assay (also called a fibrillation assay), such as that disclosed in WO2012168430.

According to an embodiment, the short acting amylin RA has a physical stability of about 20 hours or greater in a fibrillogenesis assay (also called a fibrillation assay).

According to an embodiment, the short acting amylin RA has a physical stability of about 15 hours or greater in a fibrillogenesis assay (also called a fibrillation assay).

According to an embodiment, the short acting amylin RA has a physical stability of about 10 hours or greater in a fibrillogenesis assay (also called a fibrillation assay).

According to an embodiment, the short acting amylin RA has an IC50 in a human amylin receptor potency assay, such as that disclosed in WO2013156594, of about 1200 pM or less.

Similarly to amylin, human GLP-1 has an extremely short half-life. Various GLP-1 derivatives, GLP-1 receptor agonists, referred to as GLP-1 RA, or GLP-1 analogs reproduce the effects of GLP-1, while having a longer half-life. One such FDA drug is Byetta^{®} (exenatide) which is a synthetic product of exendin-4.

GLP-1 is also described to play a similar role to that of amylin as a meal-induced inhibitory signal.

In an embodiment, the GLP-1, GLP-1 analogs, or GLP-1 RA are "short-acting" or "prandial." "Short-acting" or "prandial" is understood to mean GLP-1, GLP-1 analogs, or GLP-1 RA of which the apparent half-life of elimination after subcutaneous injection in human is less than 8 hours, in particular less than 5 hours, preferably less than 4 hours or else less than 3 hours, such as, for example, exenatide or lixisenatide.

In an embodiment, the GLP-1, the GLP-1 analogs, or the GLP-1 RA are selected from the group consisting of exenatide (Byetta^{®}, ASTRA-ZENECA), lixisenatide (Lyxumia^{®}, SANOFI), the analogs or derivatives thereof and pharmaceutically acceptable salts thereof.

Exenatide is a peptide having the sequence:
HGEGTFTSDL SKQMEEEAVR LFIEWLKNGG PSSGAPPPS (SEQ ID NO: 37)

Lixisenatide is a peptide having the sequence:
HGEGTFTSDL SKQMEEEAVR LFIEWLKNGG PSSGAPPSKK KKKK-[NH₂] (SEQ ID NO: 38)

Exenatide and lixisenatide, are described in the applications US2004/0023871 and WO0104156, entirely included herein by reference.

In an embodiment, the GLP-1, the GLP-1 analog, or GLP-1 RA is exenatide or Byetta^{®}, analogs or derivatives thereof and pharmaceutically acceptable salts thereof.

In an embodiment, the GLP-1, the GLP-1 analog, or GLP-1 RA is lixisenatide or Lyxumia^{®}, analogs or derivatives thereof and pharmaceutically acceptable salts thereof.

In an embodiment the invention concerns, a composition comprising pharmaceutical active ingredients (API) chosen in the group consisting of a short acting amylin RA chosen amongst the peptides according to Formula 1. and a GLP-1 receptor agonist (GLP-1 RA).

In an embodiment the invention relates to a composition, characterized in that it comprises only a short acting amylin RA chosen amongst the peptides according to Formula 1. and GLP-1 RA as API.

In an embodiment the invention relates to a composition, characterized in that it comprises only a short acting amylin RA chosen amongst the peptides according to Formula 1. and exenatide as API.

In an embodiment the invention relates to a composition, characterized in that it comprises only a short acting amylin RA chosen amongst the peptides according to Formula 1. and lixisenatide as API.

In an embodiment the invention concerns a composition characterized in that the GLP-1 receptor agonist (GLP-1 RA) is chosen amongst lixisenatide and exenatide.

In one embodiment the GLP-1 receptor agonist (GLP-1 RA) is lixisenatide.

In one embodiment the GLP-1 receptor agonist (GLP-1 RA) is exenatide.

The invention also relates to a composition comprising pharmaceutical active ingredients (API) chosen in the group consisting of a short acting amylin RA chosen amongst the peptides according to formula 1 and exenatide.

The invention also relates to a composition comprising pharmaceutical active ingredients (API) chosen in the group consisting of a short acting amylin RA chosen amongst the peptides according to formula 1. and lixisenatide.

In an embodiment, the composition comprises a short acting amylin RA according to formula 1 wherein Z is according to formula A1, in particular with R₁ being H and R₂ being NH₂, and a short acting GLP-1 RA, such as exenatide.

In an embodiment, the composition comprises a short acting amylin RA according to formula 1 wherein Z is according to formula B1, in particular with R₁ being H and R₂ being NH₂, and a short acting GLP-1 RA, such as exenatide.

In an embodiment, the composition comprises a short acting amylin RA according to formula 1 wherein Z is according to formula C1, in particular with R₁ being H and R₂ being NH₂, and a short acting GLP-1 RA, such as exenatide.

In an embodiment, the composition comprises a short acting amylin RA according to formula 1 wherein Z is according to formula D1, in particular with R₁ being H and R₂ being NH₂, and a short acting GLP-1 RA, such as exenatide.

In an embodiment, the composition comprises a short acting amylin RA according to formula 1 wherein Z is according to formula E1, in particular with R₁ being H and R₂ being NH₂, and a short acting GLP-1 RA, such as exenatide.

In an embodiment, the composition comprises a short acting amylin RA according to formula 1 wherein Z is according to formula A1, in particular with R₁ being H and R₂ being NH₂, and a short acting GLP-1 RA, such as lixisenatide.

In an embodiment, the composition comprises a short acting amylin RA according to formula 1 wherein Z is according to formula B1, in particular with R₁ being H and R₂ being NH₂, and a short acting GLP-1 RA, such as lixisenatide.

In an embodiment, the composition comprises a short acting amylin RA according to formula 1 wherein Z is according to formula C1, in particular with R₁ being H and R₂ being NH₂, and a short acting GLP-1 RA, such as lixisenatide.

In an embodiment, the composition comprises a short acting amylin RA according to formula 1 wherein Z is according to formula D1, in particular with R₁ being H and R₂ being NH₂, and a short acting GLP-1 RA, such as lixisenatide.

In an embodiment, the composition comprises a short acting amylin RA according to formula 1 wherein Z is according to formula E1, in particular with R₁ being H and R₂ being NH₂, and a short acting GLP-1 RA, such as lixisenatide.

In an embodiment, the composition comprises a short acting amylin RA according to formula 1 wherein Z is according to formula A1-1, in particular with R₁ being H and R₂ being NH₂, and a short acting GLP-1 RA, such as exenatide.

In an embodiment, the composition comprises a short acting amylin RA according to formula 1 wherein Z is according to formula A1-2, in particular with R₁ being H and R₂ being NH₂, and a short acting GLP-1 RA, such as exenatide.

In an embodiment, the composition comprises a short acting amylin RA according to formula 1 wherein Z is according to formula A1-3, in particular with R₁ v H and R₂ being NH₂, and a short acting GLP-1 RA, such as exenatide.

In an embodiment, the composition comprises a short acting amylin RA according to formula 1 wherein Z is according to formula A1-4, in particular with R₁ being H and R₂ being NH₂, and a short acting GLP-1 RA, such as exenatide.

In an embodiment, the composition comprises a short acting amylin RA according to formula 1 wherein Z is according to formula B1-1, in particular with R₁ being H and R₂ being NH₂, and a short acting GLP-1 RA, such as exenatide.

In an embodiment, the composition comprises a short acting amylin RA according to formula 1 wherein Z is according to formula B1-2, in particular with R₁ being H and R₂ being NH₂, and a short acting GLP-1 RA, such as exenatide.

In an embodiment, the composition comprises a short acting amylin RA according to formula 1 wherein Z is according to formula B1-3, in particular with R₁ being H and R₂ being NH₂, and a short acting GLP-1 RA, such as exenatide.

In an embodiment, the composition comprises a short acting amylin RA according to formula 1 wherein Z is according to formula C1-1, in particular with R₁ being H and R₂ being NH₂, and a short acting GLP-1 RA, such as exenatide.

In an embodiment, the composition comprises a short acting amylin RA according to formula 1 wherein Z is according to formula C1-2, in particular with R₁ being H and R₂ being NH₂, and a short acting GLP-1 RA, such as exenatide.

In an embodiment, the composition comprises a short acting amylin RA according to formula 1 wherein Z is according to formula C1-3, in particular with R₁ being H and R2 being NH₂, and a short acting GLP-1 RA, such as exenatide.

In an embodiment, the composition comprises a short acting amylin RA according to formula 1 wherein Z is according to formula C1-4, in particular with R1 being H and R₂ being NH₂, and a short acting GLP-1 RA, such as exenatide.

In an embodiment, the composition comprises a short acting amylin RA according to formula 1 wherein Z is according to formula C1-5, in particular with R₁ being H and R₂ being NH₂, and a short acting GLP-1 RA, such as exenatide.

In an embodiment, the composition comprises a short acting amylin RA according to formula 1 wherein Z is according to formula D1-1, in particular with R₁ being H and R₂ being NH₂, and a short acting GLP-1 RA, such as exenatide.

In an embodiment, the composition comprises a short acting amylin RA according to formula 1 wherein Z is according to formula D1-2, in particular with R₁ being H and R₂ being NH₂, and a short acting GLP-1 RA, such as exenatide.

In an embodiment, the composition comprises a short acting amylin RA according to formula 1 wherein Z is according to formula D1-3, in particular with R₁ being H and R₂ being NH₂, and a short acting GLP-1 RA, such as exenatide.

In an embodiment, the composition comprises a short acting amylin RA according to formula 1 wherein Z is according to formula E1-1, in particular with R₁ being H and R₂ being NH₂, and a short acting GLP-1 RA, such as exenatide.

In an embodiment, the composition comprises a short acting amylin RA according to formula 1 wherein Z is according to formula E1-2, in particular with R₁ being H and R₂ being NH₂, and a short acting GLP-1 RA, such as exenatide.

In an embodiment, the composition comprises a short acting amylin RA according to formula 1 wherein Z is according to formula A1-1, in particular with R₁ being H and R₂ being NH₂, and a short acting GLP-1 RA, such as lixisenatide.

In an embodiment, the composition comprises a short acting amylin RA according to formula 1 wherein Z is according to formula A1-2, in particular with R₁ being H and R₂ being NH₂, and a short acting GLP-1 RA, such as lixisenatide.

In an embodiment, the composition comprises a short acting amylin RA according to formula 1 wherein Z is according to formula A1-3, in particular with R₁ being H and R₂ being NH₂, and a short acting GLP-1 RA, such as lixisenatide.

In an embodiment, the composition comprises a short acting amylin RA according to formula 1 wherein Z is according to formula A1-4, in particular with R₁ being H and R₂ being NH₂, and a short acting GLP-1 RA, such as lixisenatide.

In an embodiment, the composition comprises a short acting amylin RA according to formula 1 wherein Z is according to formula B1-1, in particular with R₁ being H and R₂ being NH₂, and a short acting GLP-1 RA, such as lixisenatide.

In an embodiment, the composition comprises a short acting amylin RA according to formula 1 wherein Z is according to formula B1-2, in particular with R₁ being H and R₂ being NH₂, and a short acting GLP-1 RA, such as lixisenatide.

In an embodiment, the composition comprises a short acting amylin RA according to formula 1 wherein Z is according to formula B1-3, in particular with R₁ being H and R₂ being NH₂, and a short acting GLP-1 RA, such as lixisenatide.

In an embodiment, the composition comprises a short acting amylin RA according to formula 1 wherein Z is according to formula C1-1, in particular with R₁ being H and R₂ being NH₂, and a short acting GLP-1 RA, such as lixisenatide.

In an embodiment, the composition comprises a short acting amylin RA according to formula 1 wherein Z is according to formula C1-2, in particular with R₁ being H and R₂ being NH₂, and a short acting GLP-1 RA, such as lixisenatide.

In an embodiment, the composition comprises a short acting amylin RA according to formula 1 wherein Z is according to formula C1-3, in particular with R₁ being H and R₂ being NH₂, and a short acting GLP-1 RA, such as lixisenatide.

In an embodiment, the composition comprises a short acting amylin RA according to formula 1 wherein Z is according to formula C1-4, in particular with R₁ being H and R₂ being NH₂, and a short acting GLP-1 RA such as lixisenatide.

In an embodiment, the composition comprises a short acting amylin RA according to formula 1 wherein Z is according to formula C1-5, in particular with R₁ being H and R₂ being NH₂, and a short acting GLP-1 RA, such as lixisenatide.

In an embodiment, the composition comprises a short acting amylin RA according to formula 1 wherein Z is according to formula D1-1, in particular with R₁ being H and R₂ being NH₂, and a short acting GLP-1 RA, such as lixisenatide.

In an embodiment, the composition comprises a short acting amylin RA according to formula 1 wherein Z is according to formula D1-2, in particular with R₁ being H and R₂ being NH₂, and a short acting GLP-1 RA, such as lixisenatide.

In an embodiment, the composition comprises a short acting amylin RA according to formula 1 wherein Z is according to formula D1-3, in particular with R₁ being H and R₂ being NH2, and a short acting GLP-1 RA, such as lixisenatide.

In an embodiment, the composition comprises a short acting amylin RA according to formula 1 wherein Z is according to formula E1-1, in particular with R₁ being H and R₂ being NH₂, and a short acting GLP-1 RA, such as lixisenatide.

In an embodiment, the composition comprises a short acting amylin RA according to formula 1 wherein Z is according to formula E1-2, in particular with R₁ being H and R₂ being NH₂, and a short acting GLP-1 RA, such as lixisenatide.

In an embodiment, the composition comprises a short acting amylin RA that is not native human amylin.

In an embodiment, the composition comprises a short acting amylin RA that is not pramlintide.

In an embodiment the composition comprises a short acting amylin RA that has a sequence corresponding to [Glu14,His17,His35]-pramlintide (Cpd 1) and exenatide.

In an embodiment the composition comprises a short acting amylin RA that has a sequence corresponding to [Glu14,His17,His35]-pramlintide (Cpd 1) and lixisenatide.

In an embodiment the composition comprises a short acting amylin RA that has a sequence corresponding to [Glu14,Arg17,His35]-pramlintide (Cpd 2) and exenatide.

In an embodiment the composition comprises a short acting amylin RA that has a sequence corresponding to [Glu14,Arg17,His35]-pramlintide (Cpd 2) and lixisenatide.

In an embodiment the composition comprises a short acting amylin RA that has a sequence corresponding to [Glu14,His17,Pro37]-pramlintide (Cpd 3) and exenatide.

In an embodiment the composition comprises a short acting amylin RA that has a sequence corresponding to [Glu14,His17,Pro37]-pramlintide (Cpd 3) and lixisenatide.

In an embodiment the composition comprises a short acting amylin RA that has a sequence corresponding to [Arg1,Glu14,His17,Pro37]-pramlintide (Cpd 4) and exenatide.

In an embodiment the composition comprises a short acting amylin RA that has a sequence corresponding to [Arg1,Glu14,His17,Pro37]-pramlintide (Cpd 4) and lixisenatide.

In an embodiment the composition comprises a short acting amylin RA that has a sequence corresponding to [Glu14,Arg17,Pro37]-pramlintide (Cpd 5) and exenatide.

In an embodiment the composition comprises a short acting amylin RA that has a sequence corresponding to [Glu14,Arg17,Pro37]-pramlintide (Cpd 5) and lixisenatide.

In an embodiment the composition comprises a short acting amylin RA has the sequence EASELSTAALGRLSAELHELATLPRTETGPESP-NH₂ (SEQ ID NO: 16) (Cpd 6) and exenatide.

In an embodiment the composition comprises a short acting amylin RA has the sequence EASELSTAALGRLSAELHELATLPRTETGPESP-NH₂ (SEQ ID NO: 16) (Cpd 6) and lixisenatide.

In an embodiment the composition comprises a short acting amylin RA that has the following sequence RC()NTATC()ATQRLAEFLHRSSF-Gly(Me)-A-lle(Me)-LSSTEVGSETP-NH₂ (SEQ ID NO: 24) (Cpd 7) and exenatide.

In an embodiment the composition comprises a short acting amylin RA that has the following sequence RC()NTATC()ATQRLAEFLHRSSF-Gly(Me)-A-lle(Me)-LSSTEVGSETP-NH₂ (SEQ ID NO: 24) (Cpd 7) and lixisenatide.

In an embodiment the composition comprises a short acting amylin RA that has the following sequence R-C()-GTAT-C()-ATERLAHFLQRSSF-Gly(Me)-A-lle(Me)-LSSTEVGSNT-Hyp-NH₂ (SEQ ID NO: 25) (Cpd 8) and exenatide.

In an embodiment the composition comprises a short acting amylin RA that has the following sequence R-C()-GTAT-C()-ATERLAHFLQRSSF-Gly(Me)-A-lle(Me)-LSSTEVGSNT-Hyp-NH₂ (SEQ ID NO: 25) (Cpd 8) and lixisenatide.

In an embodiment the composition comprises a short acting amylin RA that has the following sequence RD()GTATK()ATQRLADFLQRSSF-Gly(Me)-A-lle(Me)-LSSTE-VGSNT-Hyp-NH₂ (SEQ ID NO: 29) (Cpd 9) and exenatide.

In an embodiment the composition comprises a short acting amylin RA that has the following sequence, RD()GTATK()ATERLA-Aad-FLQRSSF-Gly(Me)-A-lle(Me)-LSSTEVGSNT-Hyp-NH₂ (SEQ ID NO: 32) (Cpd 10) and exenatide.

In an embodiment the composition comprises a short acting amylin RA that has the following sequence, RD()GTATK()ATERLA-Aad-FLQRTTF-Gly(Me)-A-lle(Me)-LSSTEVGSNT-Hyp-NH2 (SEQ ID NO: 33) (Cpd 11) and exenatide.

In an embodiment the composition comprises a short acting amylin RA that has the following sequence, RD()GTATK()ATERLAHFLQRSSF-Gly(Me)-A-lle(Me)-LSSTE-VGSNT-Hyp-NH₂ (SEQ ID NO: 34) (Cpd 12) and exenatide.

In an embodiment the composition comprises a short acting amylin RA that has the following sequence, KD()GTATK()ATQRLA-Aad-FLQRSSF-Gly(Me)-Alle(Me)-LSSTEVGSNTHyp-NH₂ (SEQ ID NO: 35) (Cpd 13) and exenatide.

In an embodiment the composition comprises a short acting amylin RA that has the following sequence, KD()GTATK()ATQRLA-Aad-FLQRSSF-Gly(Me)-Alle(Me)-LSSTEVGSNTHyp-NH₂ (SEQ ID NO: 36) (Cpd 14) and exenatide.

In an embodiment the composition comprises a short acting amylin RA that has the following sequence RD()GTATK()ATQRLADFLQRSSF-Gly(Me)-A-lle(Me)-LSSTE-VGSNT-Hyp-NH₂ (SEQ ID NO: 29) (Cpd 9) and lixisenatide.

In an embodiment the composition comprises a short acting amylin RA that has the following sequence, RD()GTATK()ATERLA-Aad-FLQRSSF-Gly(Me)-A-lle(Me)-LSSTEVGSNT-Hyp-NH₂ (SEQ ID NO: 32) (Cpd 10) and lixisenatide.

In an embodiment the composition comprises a short acting amylin RA that has the following sequence, RD()GTATK()ATERLA-Aad-FLQRTTF-Gly(Me)-A-lle(Me)-LSSTEVGSNT-Hyp-NH₂ (SEQ ID NO: 33) (Cpd 11) and lixisenatide.

In an embodiment the composition comprises a short acting amylin RA that has the following sequence, RD()GTATK()ATERLAHFLQRSSF-Gly(Me)-A-lle(Me)-LSSTE-VGSNT-Hyp-NH₂ (SEQ ID NO: 34) (Cpd 12) and lixisenatide.

In an embodiment the composition comprises a short acting amylin RA that has the following sequence, KD()GTATK()ATQRLA-Aad-FLQRSSF-Gly(Me)-Alle(Me)-LSSTEVGSNTHyp-NH₂ (SEQ ID NO: 35) (Cpd 13) and lixisenatide.

In an embodiment the composition comprises a short acting amylin RA that has the following sequence, KD()GTATK()ATQRLA-Aad-FLQRSSF-Gly(Me)-Alle(Me)-LSSTEVGSNTHyp-NH₂ (SEQ ID NO: 36) (Cpd 14) and lixisenatide.

In an embodiment, the composition comprises a short acting amylin RA according to formula 1 and does not comprise a GLP-1 RA such as exenatide or lixisenatide for use for the treatment of obesity, overweight, metabolic diseases and/or diabetes, in particular type 2 diabetes.

In an embodiment, the composition comprises a short acting amylin RA according to formula 1 wherein Z is according to formula A1, B1, C1, D1 or E1 and does not comprise a GLP-1 RA such as exenatide or lixisenatide for use for the treatment of obesity, overweight, metabolic diseases and/or diabetes, in particular type 2 diabetes.

The invention also relates to stable pharmaceutical formulations including said compositions.

In an embodiment the invention relates to liquid compositions.

In an embodiment the invention relates to compositions in the form of aqueous solutions.

In an embodiment the invention relates to compositions in the form of an injectable aqueous solution.

In an embodiment the invention relates to compositions in the form of an injectable aqueous formulation.

Such formulations are typically solution or suspension.

In a further embodiment of the invention the pharmaceutical formulations are aqueous solutions.

The term "aqueous formulation" or "aqueous composition" are defined as a formulation or a composition comprising at least 50 % w/w water.

Likewise, the term "aqueous solution" is defined as a solution comprising at least 50 %w/w water, and the term "aqueous suspension" is defined as a suspension comprising at least 50 %w/w water.

The compositions in the form of injectable aqueous solutions according to the invention are clear solutions. "Clear solution" is understood to mean compositions which satisfy the criteria described in the American and European pharmacopoeias concerning the injectable solutions. In the US pharmacopoeia, the solutions are defined in part <1151> referring to the injection (<1>) (referring to <788> according to USP 35 and specified in <788> according to USP 35 and in <787>, <788> and <790> USP 38 (from August 1, 2014), according to USP 38). In the European pharmacopoeia, the injectable solutions have to meet the criteria given in sections 2.9.19 and 2.9.20.

In an embodiment the invention relates to composition, in the form of solution comprising an aprotic polar solvent.

In an embodiment the solutions comprise at least 60 % of an aprotic polar solvent.

In an embodiment the solutions comprise at least 75 % of an aprotic polar solvent.

In an embodiment the solutions comprise less than 25 % of water.

In an embodiment the solutions comprise less than 20 % of water.

In an embodiment the solutions comprise less than 15 % of water.

In an embodiment the solutions comprise less than 10 % of water.

In an embodiment the solutions comprise less than 5 % of water.

In an embodiment the solutions comprise an aprotic polar solvent chosen from a group consisting of dimethyl sulfoxide (DMSO), dimethylformamide (DMF), ethyl acetate, n-methyl pyrrolidone (NMP), dimethylacetamide (DMA), and propylene carbonate.

In an embodiment, the solutions comprise dimethyl sulfoxide (DMSO).

In an embodiment, the solutions comprise as solvent dimethyl sulfoxide (DMSO) and less than 25 % of water.

In an embodiment the invention relates to composition in solid form.

In an embodiment the invention relates to compositionsin powder form.

In an embodiment the invention relates to dried-form compositions.

In an embodiment the invention relates to spray-dried compositions.

In an embodiment the invention relates to freeze-dried compositions.

In one embodiment the physician or the patient adds solvents and/or diluents prior to use.

In another embodiment the pharmaceutical formulations are in the form of dried form formulations (e.g. freeze-dried or spray-dried) ready for use without any prior dissolution.

By "dried form" is intended the liquid pharmaceutical composition or formulation is dried either by freeze-drying (i.e., lyophilization), spray-drying or air-drying.

If the compositions are in the form of solutions the contents of the different constituents are given in in weight per volume.

If the compositions are in the form of solid compositions the contents of the different constituents are given in weight per weight.

In an embodiment the invention relates to compositions, wherein the pH is ranging from 3.0 to 5.0.

In an embodiment the invention relates to compositions, wherein the pH is ranging from 3.5 to 4.4.

In an embodiment the invention relates to compositions, wherein the pH of the solution is comprised from 3.5 to 4.4.

In an embodiment the invention relates to compositions, wherein the pH of the solution is comprised from 3.7 to 4.2.

In an embodiment the invention relates to compositions, wherein the pH of the solution is about 4.0.

In an embodiment the invention relates to compositions, wherein the pH is ranging from 6.0 to 8.0.

In an embodiment the invention relates to compositions, wherein the pH is ranging from 6.2 to 7.8.

In an embodiment the invention relates to compositions, wherein the pH is ranging from 6.4 to 7.6.

In an embodiment the invention relates to compositions, wherein the pH of the solution is ranging from 6.6 to 7.4.

In an embodiment the invention relates to compositions, wherein the pH of the solution is ranging from 6.8 to 7.4.

The invention also relates to compositions according to the invention which is intended to be used in the treatment of metabolic diseases.

The invention also relates to compositions according to the invention which is intended to be used in an obesity treatment method, characterized in that it enables to decrease the food consumption. Obese person being defined as person having a BMI (body mass index) of 30 or higher.

The invention also relates to compositions according to the invention which is intended to be used in an overweight treatment method, characterized in that it enables to decrease the food consumption. Overweight person being defined as person having a BMI from 25 to less than 30.

In an embodiment the compositions according to the invention are intended to be used in an overweight or obesity treatment method for person having a BMI of 25 or higher.

In an embodiment the compositions according to the invention are intended to be used in an overweight or obesity treatment method for person having a BMI of 27 or higher.

The invention also relates to compositions according to the invention which are intended to be used in method for the treatment of diabetes, characterized in that it enables to decrease the food consumption.

The invention also relates to compositions according to the invention which are intended to be used in an obesity treatment method, characterized in that it improves loss of weight or limit the gain of weight.

In an embodiment, the compositions according to the invention lead to a slow-down of the gastric emptying of at least 5 % inferior to an identical composition without the corresponding GLP-1 RA.

In one embodiment, the composition comprises a short acting amylin RA chosen amongst the peptides according to Formula 1 at a concentration ranging from 0.3 to 10 mg/ml.

In one embodiment, the compositions comprise a short acting amylin RA chosen amongst the peptides according to formula 1 at a concentration ranging from 0.5 to 10 mg/ml.

In one embodiment, the compositions comprise a short acting amylin RA chosen amongst the peptides according to formula 1 at a concentration ranging from 0.6 to 6 mg/ml.

In one embodiment, the compositions comprise a short acting amylin RA chosen amongst the peptides according to formula 1 at a concentration ranging from 0.9 to 4 mg/ml.

In one embodiment, the compositions comprise a short acting amylin RA chosen amongst the peptides according to formula 1 at a concentration ranging from 1 to 4 mg/ml.

In one embodiment, the compositions comprise s a short acting amylin RA chosen amongst the peptides according to formula 1 at a concentration ranging from 1 to 3 mg/ml.

In one embodiment, the compositions comprise a short acting amylin RA chosen amongst the peptides according to formula 1 at a concentration ranging from 1 to 2 mg/ml.

In one embodiment, the short acting amylin RA chosen amongst the peptides according to formula 1 concentration is 0.6 mg/ml.

In one embodiment the short acting amylin RA chosen amongst the peptides according to formula 1 concentration is 1 mg/ml.

In one embodiment, the short acting amylin RA chosen amongst the peptides according to formula 1 concentration is 1.5 mg/ml.

In one embodiment, the short acting amylin RA chosen amongst the peptides according to formula 1 concentration is 2 mg/ml.

In one embodiment, the short acting amylin RA chosen amongst the peptides according to formula 1 concentration is 2.5 mg/ml.

In one embodiment, the short acting amylin RA chosen amongst the peptides according to formula 1 concentration is 3 mg/ml.

In one embodiment, the short acting amylin RA chosen amongst the peptides according to formula 1 concentration is 3.5 mg/ml.

In one embodiment, the short acting amylin RA chosen amongst the peptides according to formula 1 concentration is 4 mg/ml.

In one embodiment, the short acting amylin RA chosen amongst the peptides according to formula 1 concentration is 4.5 mg/ml.

In one embodiment, the short acting amylin RA chosen amongst the peptides according to formula 1 concentration is 5 mg/ml.

In one embodiment, the compositions comprise a short acting amylin RA chosen amongst the peptides according to formula 1 in an amount less than or equal to 20 % w/w.

In one embodiment, the compositions comprise a short acting amylin RA chosen amongst the peptides according to formula 1 in an amount less than or equal to 15 % w/w.

In one embodiment, the compositions comprise a short acting amylin RA chosen amongst the peptides according to formula 1 in an amount less than or equal to 10 % w/w.

In one embodiment, the compositions comprise a short acting amylin RA chosen amongst the peptides according to formula 1e in an amount less than or equal to 5 % w/w.

In one embodiment, the compositions comprise a short acting amylin RA chosen amongst the peptides according to formula 1 in an amount comprised from 1 to 20 % w/w.

In one embodiment, the compositions comprise a short acting amylin RA chosen amongst the peptides according to formula 1 in an amount comprised from 2 to 15 % w/w.

In one embodiment, the compositions comprise exenatide at a concentration ranging from 20 to 500 µg/ml.

In one embodiment, the compositions comprise exenatide at a concentration ranging from 20 to 200 µg/ml

In one embodiment, the compositions comprise s exenatide at a concentration ranging from 60 to 400 µg/ml.

In one embodiment, the compositions comprise exenatide at a concentration ranging from 80 to 300 µg/ml.

In one embodiment, the compositions comprise exenatide at a concentration ranging from 90 to 250 µg/ml.

In one embodiment, the compositions comprise exenatide at a concentration ranging from 100 to 200 µg/ml.

In one embodiment the exenatide concentration is 50 µg/ml.

In one embodiment the exenatide concentration is 75 µg/ml.

In one embodiment the exenatide concentration is 100 µg/ml.

In one embodiment the exenatide concentration is 125 µg/ml.

In one embodiment the exenatide concentration is 150 µg/ml.

In one embodiment the exenatide concentration is 175 µg/ml.

In one embodiment the exenatide concentration is 200 µg/ml.

In one embodiment the exenatide concentration is 250 µg/ml.

In one embodiment the exenatide concentration is 300 µg/ml.

In one embodiment the exenatide concentration is 350 µg/ml.

In one embodiment the exenatide concentration is 400 µg/ml.

In one embodiment the exenatide concentration is 450 µg/ml.

In one embodiment the exenatide concentration is 500 µg/ml.

In one embodiment, the compositions comprise exenatide in an amount less than or equal to 10 % w/w.

In one embodiment, the compositions comprise exenatide in an amount less than or equal to 7.5 % w/w.

In one embodiment, the compositions comprise exenatide in an amount less than or equal to 5 % w/w.

In one embodiment, the compositions comprise exenatide in an amount less than or equal to 2.5 % w/w.

In one embodiment, the compositions comprise lixisenatide at a concentration ranging from 50 to 1250 µg/ml.

In one embodiment, the compositions comprise lixisenatide at a concentration ranging from 125 to 1000 µg/ml.

In one embodiment, the compositions comprise lixisenatide at a concentration ranging from 150 to 1000 µg/ml.

In one embodiment, the compositions comprise lixisenatide at a concentration ranging from 200 to 750 µg/ml.

In one embodiment, the compositions comprise lixisenatide at a concentration ranging from 225 to 650 µg/ml.

In one embodiment, the compositions comprise lixisenatide at a concentration ranging from 250 to 500 µg/ml.

In one embodiment the lixisenatide concentration is 150 µg/ml.

In one embodiment the lixisenatide concentration is 200 µg/ml.

In one embodiment the lixisenatide concentration is 250 µg/ml.

In one embodiment the lixisenatide concentration is 300 µg/ml.

In one embodiment the lixisenatide concentration is 350 µg/ml.

In one embodiment the lixisenatide concentration is 400 µg/ml.

In one embodiment the lixisenatide concentration is 450 µg/ml.

In one embodiment the lixisenatide concentration is 500 µg/ml.

In one embodiment the lixisenatide concentration is 550 µg/ml.

In one embodiment the lixisenatide concentration is 600 µg/ml.

In one embodiment the lixisenatide concentration is 650 µg/ml.

In one embodiment the lixisenatide concentration is 700 µg/ml.

In one embodiment the lixisenatide concentration is 750 µg/ml.

In one embodiment the lixisenatide concentration is 800 µg/ml.

In one embodiment, the compositions comprise lixisenatide in an amount less than or equal to 30 % w/w.

In one embodiment, the compositions comprise lixisenatide in an amount less than or equal to 20 % w/w.

In one embodiment, the compositions comprise lixisenatide in an amount less than or equal to 15 % w/w.

In one embodiment, the compositions comprise lixisenatide in an amount less than or equal to 7.5 % w/w.

In one embodiment, the composition is characterized in that the ratio short acting amylin RA chosen amongst the peptides according to Formula 1 / exenatide (weight/weight) is ranging from 2 to 50.

In one embodiment, the composition is characterized in that the ratio short acting amylin RA chosen amongst the peptides according to formula 1 / exenatide (weight/weight) is ranging from 2 to 30.

In one embodiment, the composition is characterized in that the ratio short acting amylin RA chosen amongst the peptides according to formula 1 / exenatide (weight/weight) is ranging from 3 to 25.

In one embodiment, the composition is characterized in that the ratio short acting amylin RA chosen amongst the peptides according to formula 1 / exenatide (weight/weight) is ranging from 5 to 20.

In one embodiment, the composition is characterized in that the ratio short acting amylin RA chosen amongst the peptides according to formula 1 / exenatide (weight/weight) is ranging from 8 to 15.

In one embodiment, the composition is characterized in that it comprises from 20 to 400 µg of exenatide per mg of short acting amylin RA chosen amongst the peptides according to formula 1.

In one embodiment, the composition is characterized in that it comprises from 30 to 200 µg of exenatide per mg of short acting amylin RA chosen amongst the peptides according to formula 1.

In one embodiment, the composition is characterized in that it comprises from 40 to 150 µg of exenatide per mg of short acting amylin RA chosen amongst the peptides according to formula 1.

In one embodiment, the composition is characterized in that it comprises from 40 to 120 µg of exenatide per mg of short acting amylin RA chosen amongst the peptides according to formula 1.

In one embodiment, the composition is characterized in that the ratio short acting amylin RA chosen amongst the peptides according to formula 1 / lixisenatide (weight/weight) is ranging from 0.7 to 20.

In one embodiment, the composition is characterized in that the ratio short acting amylin RA chosen amongst the peptides according to formula 1 / lixisenatide (weight/weight) is ranging from 0.7 to 15.

In one embodiment, the composition is characterized in that the ratio short acting amylin RA chosen amongst the peptides according to formula 1 / lixisenatide (weight/weight) is ranging from 1.2 to 8.

In one embodiment, the composition is characterized in that the ratio short acting amylin RA chosen amongst the peptides according to formula 1 / lixisenatide (weight/weight) is ranging from 1.8 to 7.

In one embodiment, the composition is characterized in that the ratio short acting amylin RA chosen amongst the peptides according to formula 1 / lixisenatide (weight/weight) is ranging from 2.5 to 6.

In one embodiment, the composition is characterized in that it comprises from 50 to 1000 µg of lixisenatide per mg of short acting amylin RA chosen amongst the peptides according to formula 1.

In one embodiment, the composition is characterized in that it comprises from 75 to 500 µg of lixisenatide per mg of short acting amylin RA chosen amongst the peptides according to formula 1.

In one embodiment, the composition is characterized in that it comprises from 100 to 375 µg of lixisenatide per mg of short acting amylin RA chosen amongst the peptides according to formula 1.

In one embodiment, the composition is characterized in that it comprises from 100 to 300 µg of lixisenatide per mg of short acting amylin RA chosen amongst the peptides according to formula 1.

The invention also concerns at least two compositions, at least one of these comprising a short acting amylin RA and doesn't comprise any GLP-1 RA and at least one of these comprising a GLP-1 RA and does not comprise any amylin RA for use to reduce the slow-down of gastric emptying, said compositions are co-administered in a therapeutical scheme that comprises simultaneous or sequential administration characterized in that the ratio short acting amylin RA chosen amongst the peptides according to formula 1 / exenatide (weight/weight) is ranging from 2 to 50..

The invention also concerns at least two compositions, at least one of these comprising a short acting amylin RA and doesn't comprise any GLP-1 RA and at least one of these comprising a GLP-1 RA for use for the treatment of obesity, said compositions are co-administered in a therapeutical scheme that comprises simultaneous or sequential administration characterized in that the ratio short acting amylin RA chosen amongst the peptides according to formula 1/ exenatide (weight/weight) is ranging from 2 to 50.

The invention also concerns at least two compositions, at least one of these comprising a short acting amylin RA and doesn't comprise any GLP-1 RA and at least one of these comprising a GLP-1 RA for use for the treatment of metabolic diseases, said compositions are co-administered in a therapeutical scheme that comprises simultaneous or sequential administration characterized in that the ratio short acting amylin RA chosen amongst the peptides according to formula 1/ exenatide (weight/weight) is ranging from 2 to 50.

The invention also concerns at least two compositions, at least one of these comprising a short acting amylin RA and doesn't comprise any GLP-1 RA and at least one of these comprising a GLP-1 RA for use for the treatment of obesity, overweight, metabolic diseases and/or diabetes, in particular type 2 diabetes, said compositions are co-administered in a therapeutical scheme that comprises simultaneous or sequential administration characterized in that the ratio short acting amylin RA chosen amongst the peptides according to formula 1 / exenatide (weight/weight) is ranging from 2 to 50..

In one embodiment, the invention relates to a method for reducing the slow-down of gastric emptying, wherein at least two compositions comprising at least one short acting amylin RA and does not comprise a GLP-1 RA, and at least one short acting GLP-1 RA, such as exenatide or lixisenatide, and does not comprise an amylin RA, are co-administered in a therapeutical scheme that comprises simultaneous or sequential administration, characterized in that the ratio short acting amylin RA chosen amongst the peptides according to formula 1 / exenatide (weight/weight) is ranging from 2 to 50.

In one embodiment, the invention relates to a method for the treatment of obesity, wherein at least two compositions comprising at least one short acting amylin RA and does not comprise a GLP-1 RA, and at least one short acting GLP-1 RA, such as exenatide or lixisenatide, and does not comprise an amylin RA, are co-administered in a therapeutical scheme that comprises simultaneous or sequential administration, characterized in that the ratio short acting amylin RA chosen amongst the peptides according to formula 1 / exenatide (weight/weight) is ranging from 2 to 50.

In one embodiment, the invention relates to a method for the treatment of metabolic diseases, wherein at least two compositions comprising at least one short acting amylin RA and does not comprise a GLP-1 RA, and at least one short acting GLP-1 RA, such as exenatide or lixisenatide, and does not comprise an amylin RA, are co-administered in a therapeutical scheme that comprises simultaneous or sequential administration, characterized in that the ratio short acting amylin RA chosen amongst the peptides according to formula 1 / exenatide (weight/weight) is ranging from 2 to 50.

In one embodiment, the invention relates to a method for the treatment of obesity, overweight, metabolic diseases and/or diabetes, in particular type 2 diabetes, wherein at least two compositions comprising at least one short acting amylin RA and does not comprise a GLP-1 RA, and at least one short acting GLP-1 RA, such as exenatide or lixisenatide, and does not comprise an amylin RA, are co-administered in a therapeutical scheme that comprises simultaneous or sequential administration, characterized in that the ratio short acting amylin RA chosen amongst the peptides according to formula 1 / exenatide (weight/weight) is ranging from 2 to 50.

In an embodiment the method is characterized in that the short acting amylin RA is not native human amylin or pramlintide

In one embodiment, the ratio short acting amylin RA chosen amongst the peptides according to formula 1 / exenatide (weight/weight) is ranging from 2 to 30.

In one embodiment, the ratio short acting amylin RA chosen amongst the peptides according to formula 1 / exenatide (weight/weight) is ranging from 3 to 25.

In one embodiment, the ratio short acting amylin RA chosen amongst the peptides according to formula 1 / exenatide (weight/weight) is ranging from 5 to 20.

In one embodiment, the ratio short acting amylin RA chosen amongst the peptides according to formula 1 / exenatide (weight/weight) is ranging from 8 to 15.

The invention also concerns at least two compositions, at least one of these comprising a short acting amylin RA and doesn't comprise any GLP-1 RA and at least one of these comprising a short acting GLP-1 RA and does not comprise an amylin RA for use to reduce the slow-down of gastric emptying, said compositions are co-administered in a therapeutical scheme that comprises simultaneous or sequential administration characterized in that the ratio short acting amylin RA chosen amongst the peptides according to formula 1 / lixisenatide (weight/weight) is ranging from 0.7 to 20..

The invention also concerns at least two compositions, at least one of these comprising a short acting amylin RA and doesn't comprise any GLP-1 RA and at least one of these comprising a short acting GLP-1 RA and does not comprise an amylin RA for use for the treatment of obesity, said compositions are co-administered in a therapeutical scheme that comprises simultaneous or sequential administration? characterized in that the ratio short acting amylin RA chosen amongst the peptides according to formula 1 / lixisenatide (weight/weight) is ranging from 0.7 to 20.

The invention also concerns at least two compositions, at least one of these comprising a short acting amylin RA and doesn't comprise any GLP-1 RA and at least one of these comprising a short acting GLP-1 RA and does not comprise an amylin RA for use for the treatment of obesity, overweight, metabolic diseases and/or diabetes, in particular type 2 diabetes, said compositions are co-administered in a therapeutical scheme that comprises simultaneous or sequential administration characterized in that the ratio short acting amylin RA chosen amongst the peptides according to formula 1 / lixisenatide (weight/weight) is ranging from 0.7 to 20.

In one embodiment, the invention relates to a method for reducing the slow-down of gastric emptying, wherein at least two compositions comprising at least one short acting amylin RA and does not comprise a GLP-1 RA, and at least one short acting GLP-1 RA such as exenatide or lixisenatide and does not comprise an amylin RA are co-administered in a therapeutical scheme that comprises simultaneous or sequential administration, characterized in that the ratio short acting amylin RA chosen amongst the peptides according to formula 1 / lixisenatide (weight/weight) is ranging from 0.7 to 20.

In one embodiment, the invention relates to a method for the treatment of obesity, wherein at least two compositions comprising at least one short acting amylin RA and does not comprise a GLP-1 RA , and at least one short acting GLP-1 RA such as exenatide or lixisenatide and does not comprise an amylin RA are co-administered in a therapeutical scheme that comprises simultaneous or sequential administration, characterized in that the ratio short acting amylin RA chosen amongst the peptides according to formula 1 / lixisenatide (weight/weight) is ranging from 0.7 to 20.

In one embodiment, the invention relates to a method for the treatment of obesity, overweight and/or diabetes, in particular type 2 diabetes, wherein at least two compositions comprising at least one short acting amylin RA and does not comprise a GLP-1 RA , and at least one short acting GLP-1 RA such as exenatide or lixisenatide and does not comprise an amylin RA are co-administered in a therapeutical scheme that comprises simultaneous or sequential administration, characterized in that the ratio short acting amylin RA chosen amongst the peptides according to formula 1 / lixisenatide (weight/weight) is ranging from 0.7 to 20.

In an embodiment the method is characterized in that the short acting amylin RA is not native human amylin or pramlintide

In one embodiment, the ratio short acting amylin RA chosen amongst the peptides according to formula 1 / lixisenatide (weight/weight) is ranging from 0.7 to 15.

In one embodiment, the ratio short acting amylin RA chosen amongst the peptides according to formula 1 / lixisenatide (weight/weight) is ranging from 1.2 to 8.

In one embodiment, the ratio short acting amylin RA chosen amongst the peptides according to formula 1 / lixisenatide (weight/weight) is ranging from 1.8 to 7.

In one embodiment, the ratio short acting amylin RA chosen amongst the peptides according to formula 1 / lixisenatide (weight/weight) is ranging from 2.5 to 6.

In an embodiment, the compositions according to the invention moreover include buffers.

In an embodiment, the composition according to the invention include a buffer selected from the group consisting of a sodium acetate buffer and Tris buffer.

In one embodiment, the composition are characterized in that it comprises a sodium acetate buffer.

In one embodiment, the compositions are characterized in that it comprises a Tris buffer

In an embodiment, the compositions according to the invention moreover include preservatives.

In an embodiment, the preservatives are selected from the group consisting of m-cresol and phenol, alone or in a mixture.

In an embodiment, the concentration of preservatives is from 10 to 50 mM.

In an embodiment, the concentration of preservatives is from 10 to 40 mM.

In an embodiment, the compositions according to the invention moreover include a surfactant.

In an embodiment, the surfactant is selected from the group consisting of Poloxamer 188, Tween^{®} 20, also referred to as Polysorbate 20, and Tween^{®} 80, also referred to as Polysorbate 80.

In an embodiment, the Tween^{®} 20 concentration varies from 5 to 50 µg/mL.

In an embodiment, the Tween^{®} 20 concentration varies from 5 to 25 µg/mL.

In an embodiment, the Tween^{®} 20 concentration is 10 µg/mL

In one embodiment, the compositions are characterized in that the concentration of Poloxamer 188 is ranging from 5 to 50 µg/ml.

In one embodiment, the compositions are characterized in that the concentration of Poloxamer 188 is ranging from 5 to 25 µg/ml.

In one embodiment, the compositions are characterized in that the concentration of Poloxamer 188 is 10 µg/mL.

In an embodiment the compositions further comprise an absorption enhancer.

In an embodiment, the absorption enhancer is chosen from the group consisting of SNAC, N-(10-[2-hydroxybenzoyl]amino)decanoic acid (SNAD), 8-[N-(2-hydroxy-4-methoxybenzoyl)amino]caprylic acid (4-MOAC), 8-[N-(2-hydroxy-5-chlorobenzoyl)amino]caprylic acid (5-CNAC) and 4-[(4-chloro-2-hydroxy-benzoyl)amino]butanoic acid (4-CNAB) and sodium salts thereof.

In an embodiment the absorption enhancer is SNAC (sodium 8-N-(2-hydroxybenzoyl)aminocaprylate).

In one embodiment, the compositions comprise an absorption enhancer in an amount comprised from 50 to 90 % w/w.

In one embodiment, the compositions comprise an absorption enhancer in an amount comprised from 55 to 85 % w/w.

In one embodiment, the compositions comprise an absorption enhancer in an amount comprised from 70 to 80 % w/w.

In one embodiment, the compositions comprise an absorption enhancer in an amount at least 5 % w/w

In one embodiment, the compositions comprise an absorption enhancer in an amount at least 10 % w/w

In one embodiment, the compositions comprise an absorption enhancer in an amount at least 15 % w/w

In one embodiment, the compositions comprise an absorption enhancer in an amount at least 20 % w/w

In one embodiment, the compositions comprise an absorption enhancer in an amount at least 25 % w/w

In one embodiment, composition comprise an absorption enhancer in an amount at least 30 % w/w

In one embodiment, the compositions comprise an absorption enhancer in an amount at least 35 % w/w

In one embodiment, the compositions comprise an absorption enhancer in an amount at least 40 % w/w

In one embodiment, the compositions comprise an absorption enhancer in an amount at least 45 % w/w

In one embodiment, the compositions comprise an absorption enhancer in an amount at least 50 % w/w.

In one embodiment, the compositions comprise an absorption enhancer in an amount at least 55 % w/w

In one embodiment, the compositions comprise an absorption enhancer in an amount at least 60 % w/w

The compositions according to the invention can moreover include additives such as tonicity agents.

In an embodiment, the tonicity agents are selected from the group consisting of glycerol, sodium chloride, mannitol and glycine.

In an embodiment, the compositions according to the invention further include an antioxidant.

In an embodiment, the antioxidant is methionine.

In an embodiment, the compositions according to the invention are characterized in that it does not comprise insulin.

In an embodiment, the compositions according to the invention are characterized in that it does not comprise human insulin.

In an embodiment, the compositions according to the invention are characterized in that it does not comprise insulin chosen amongst human insulin analogs.

In an embodiment, the compositions according to the invention are characterized in that it does not comprise human insulin analogs chosen amongst insulin aspart (NovoLog^{®} from NOVO NORDISK), insulin lispro (Humalog^{®} from ELI LILLY) and glulisine insulin (Apidra^{®} from SANOFI).

In an embodiment, the compositions according to the invention are characterized in that it does not comprise human insulin analogs chosen amongst insulin comprising at least the A21G mutation.

In an embodiment, the compositions according to the invention are characterized in that it does not comprise human insulin analogs chosen amongst insulin analogs A21G, B28D, desB30 and A21G, B28E, desB30 described in application WO2007104786 entirely included herein by reference.

In an embodiment, the composition according to the invention do not comprise the human insulin analog comprising only the mutation A21G.

This human insulin analog comprising only the mutation A21G has the following sequences:
- the chain A sequence is SEQ ID NO: 39 GIVEQCCTSICSLYQLENYCG and
- the chain B sequence is SEQ ID NO: 40 FVNQHLCGSHLVEALYLVCGERGFFYTPKT.

In an embodiment, the compositions according to the inventio are characterized in that it does not comprise insulin glargine.

In an embodiment, the compositions according to the invention are characterized in that it does not comprise glucagon or glucagon analogs.

In an embodiment, the compositions according to the invention do not comprise a co-polyaminoacid bearing carboxylate charges and hydrophobic radicals.

In an embodiment, the hydrophobic radicals are chosen amongst those as described in the application US2019274954A1 entirely included herein by reference.

In an embodiment, the compositions according to the invention are characterized in that it does not comprise a co-polyamino acid bearing carboxylate charges and hydrophobic radicals as described in the application US20190328842A1 entirely included herein by reference.

In an embodiment the compositions according to the invention are characterized in that it does not comprise a co-polyamino acid bearing carboxylate charges and hydrophobic radicals as described in the application US2019274954A1 entirely included herein by reference.

In an embodiment the compositions according to the invention are characterized in that it does not comprise a co-polyamino acid bearing carboxylate charges and hydrophobic radicals as described in the applications US20180193421A1 and US2019275108A1 entirely included herein by reference.

In an embodiment the compositions according to the invention are characterized in that it does not comprise an amphiphilic compound comprising a hydrophilic backbone substituted by hydrophobic radicals as described in the application PCT/EP/2019/070953 entirely included herein by reference.

The invention also concerns compositions for use in a method for the treatment of obesity.

The invention also concerns compositions for use in a method for the treatment of overweight.

The invention also concerns compositions for use in a method for the treatment of metabolic diseases.

The invention also concerns compositions for use in a method for the treatment of diabetes, in particular type 2 diabetes.

In an embodiment the method of treatment concerns animals.

In another embodiment the method of treatment concerns mammals.

In another embodiment the method of treatment concerns mammals or pets, such as dogs and cats.

In another embodiment the method of treatment concerns human beings.

In one embodiment, the compositions according to the invention for use in the method for the treatment of obesity, overweight, metabolic diseases and/or diabetes, in particular type 2 diabetes, are characterized in that it reduces the slowing effect of gastric emptying of amylin RA as compared to the same composition without GLP-1 RA.

In one embodiment, the compositions according to the invention for use in a method for the treatment of obesity, overweight, metabolic diseases and/or diabetes, in particular type 2 diabetes, are characterized in that it reduces the slowing effect on gastric emptying of amylin RA as compared to the same composition without GLP-1 RA by at least 5%.

In one embodiment, the compositions according to the invention for use in the method for the treatment of obesity, overweight, metabolic diseases and/or diabetes, in particular type 2 diabetes, are characterized in that it reduces the slowing effect on gastric emptying of amylin RA as compared to the same composition without GLP-1 RA by at least 10%.

In one embodiment, the compositions according to the invention for use in the method for the treatment of obesity, overweight, metabolic diseases and/or diabetes, in particular type 2 diabetes, are characterized in that it reduces the slowing effect on gastric emptying of amylin RA as compared to the same composition without GLP-1 RA by at least 20%.

In one embodiment, the compositions according to the invention for use in the method for the treatment of obesity, overweight, metabolic diseases and/or diabetes, in particular type 2 diabetes, are characterized in that it reduces the slowing effect on gastric emptying of amylin RA as compared to the same composition without GLP-1 RA by at least 30%.

In one embodiment, the compositions according to the invention for use in the method for the treatment of obesity, overweight, metabolic diseases and/or diabetes, in particular type 2 diabetes, are characterized in that it reduces the slowing effect on gastric emptying of amylin RA as compared to the same composition without GLP-1 RA by at least 40%.

In one embodiment, the compositions according to the invention for use in the method for the treatment of obesity, overweight, metabolic diseases and/or diabetes, in particular type 2 diabetes, are characterized in that it reduces at least one adverse effect as compared to the same composition without GLP-1 RA.

In one embodiment, the composition according to the invention for the treatment of obesity, overweight, metabolic diseases and/or diabetes, in particular Type 2 Diabetes, is characterized in that the daily dose of short acting amylin RA is ranging from 150 to 2000 µg/day.

In one embodiment, the composition according to the invention for the treatment of obesity and/or diabetes, in particular Type 2 Diabetes, is characterized in that the daily dose of short acting amylin RA is ranging from 250 to 1800 µg/day.

In one embodiment, the composition according to the invention for the treatment of obesity and/or diabetes, in particular Type 2 Diabetes, is characterized in that the daily dose of short acting amylin RA is ranging from 300 to 1500 µg/day.

In one embodiment, the composition according to the invention for the treatment of obesity and/or diabetes, in particular Type 2 Diabetes, is characterized in that the daily dose of short acting amylin RA is ranging from 350 to 1200 µg/day.

In one embodiment, the composition according to the invention for the treatment of obesity and/or diabetes, in particular Type 2 Diabetes, is characterized in that the daily dose of short acting amylin RA is ranging from 400 to 1000 µg/day.

In one embodiment, the composition according to the invention for the treatment of obesity and/or diabetes, in particular Type 2 Diabetes, is characterized in that the daily dose of short acting amylin RA is ranging from 400 to 800 µg/day.

In one embodiment, the compositions according to the invention, are characterized in that the daily dose of exenatide is ranging from 10 to 250 µg.

In one embodiment, the compositions according to the invention, are characterized in that the daily dose of exenatide is ranging from 20 to 200 µg.

In one embodiment, the compositions according to the invention, are characterized in that the daily dose of exenatide is ranging from 25 to 180 µg.

In one embodiment, the compositions according to the invention, are characterized in that the daily dose of exenatide is ranging from 30 to 160 µg.

In one embodiment, the compositions according to the invention, are characterized in that the daily dose of exenatide is ranging from 35 to 140 µg.

In one embodiment, the compositions according to the invention, are characterized in that the daily dose of exenatide is ranging from 40 to 120 µg.

In one embodiment, the compositions according to the invention, are characterized in that the daily dose of lixisenatide is ranging from 25 to 600 µg.

In one embodiment, the compositions according to the invention, are characterized in that the daily dose of lixisenatide is ranging from 50 to 500 µg.

In one embodiment, the compositions according to the invention, are characterized in that the daily dose of lixisenatide is ranging from 65 to 450 µg.

In one embodiment, the compositions according to the invention, are characterized in that the daily dose of lixisenatide is ranging from 75 to 400 µg.

In one embodiment, the compositions according to the invention, are characterized in that the daily dose of lixisenatide is ranging from 85 to 350 µg.

In one embodiment, the compositions according to the invention, are characterized in that the daily dose of lixisenatide is ranging from 95 to 300 µg.

The invention also concerns containers comprising pharmaceutical formulations according to the invention.

In one embodiment the container is a cartridge.

In one embodiment the container is a vial.

The invention also concerns an injection or delivery device comprising pharmaceutical formulations according to the invention.

In one embodiment, the injection or delivery device comprises pharmaceutical formulations according to the invention.

In one embodiment, the injection or delivery device is a pump.

In one embodiment, the injection or delivery device delivers the pharmaceutical formulation continuously.

In one embodiment, the injection or delivery device delivers microboluses of the pharmaceutical formulation.

In one embodiment, the injection or delivery device is a patch pump

In one embodiment, the injection or delivery device is an osmotic pump.

Composition according to the invention that comprises 1 mg/mL of short acting amylin RA chosen amongst the peptides according to formula 1 and 100 µg/mL of exenatide.

Composition according to the invention that comprises 1 mg/mL of short acting amylin RA chosen amongst the peptides according to formula 1 and 250 µg/mL of lixisenatide.

The compositions according to the invention can moreover include excipients in compliance with the Pharmacopoeias, in particular the EP and/or US Pharmacopoeias.

According to an embodiment, the composition can be in solid or lyophilized form. This composition can then be used to reconstitute a solution or a formulation.

The methods of administration considered are the intravenous, subcutaneous, intradermal or intramuscular route.

According to an embodiment, the method of administration is the subcutaneous route.

The transdermal, oral, nasal, vaginal, ocular, buccal, pulmonary administration routes are also considered.

The invention also relates to single-dose formulations.

In an embodiment, the formulations are in the form of an injectable solution.

The preparation of a composition according to the invention has the advantage that it can be implemented by simple mixing of an aqueous solution of short acting amylin RA chosen amongst the peptides according to formula 1 and GLP-1 RA, in aqueous solution or in lyophilized form.

If necessary, the composition of the mixture is adjusted in terms of excipients such as glycerol, m-cresol and polysorbate 20 (Tween^{®} 20). This addition can be carried out by addition of concentrated solutions of said excipients.

### Examples

### Compositions

### Example C1 : 250 µg/mL exenatide solution (Byetta^{®})

This solution is a commercial solution of exenatide marketed by the company NOVO NORDISK under the name of Byetta^{®}. The excipients in this composition C1 are detailed in Table 2.

### Example C1' : Preparation of a 2 mg/mL exenatide solution at pH 4 (Composition C1')

Exenatide powder (obtained by peptide synthesis) is dissolved with a 10 mM HCl solution. An acetate buffer solution is then added to give the composition C1'. The composition is detailed in Table 2.

### Example C2: Preparation of a 250 µg/mL pramlintide solution at pH 4 (Composition C2).

A 5 mg/mL concentrated solution of pramlintide (obtained by peptide synthesis) and excipients is prepared via a stepwise addition in an empty container in the following order: m-cresol, acetate buffer, mannitol and pramlintide. Sufficient quantity of water is then added and the pH is adjusted to 4 to obtain composition C2 which is detailed in Table 2.

### Exemple C2' : Preparation of a 10 mg/mL pramlintide solution at pH 4 (Composition C2').

Pramlintide powder (obtained by peptide synthesis) is dissolved with a 10 mM HCl solution. An acetate buffer solution is then added to give the composition C2'. The composition is detailed in Table 2.

### Exemple C2" : Preparation of 2 mg/mL short acting amylin RA compositions at pH 4 (Compositions C2").

Short acting amylin RA powder (obtained by peptide synthesis) is dissolved with a 10 mM HCl solution. An acetate buffer solution is then added to give the composition C2". The short acting amylin RA are as follows detailed in Table 1.

**Table 1 Compositions C2‴-1 to C2‴- 14**

| **Composition** | **Short acting amylin RA Formula (Cpd)** |
|---|---|
| C2"-1 | (Cpd. 1) |
| C2"-2 | (Cpd. 2) |
| C2"-3 | (Cpd. 3) |
| C2"-4 | (Cpd. 4) |
| C2"-5 | (Cpd. 5) |
| C2"-6 | (Cpd. 6) |
| C2"-7 | (Cpd. 7) |
| C2"-8 | (Cpd. 8) |
| C2"-9 | (Cpd. 9) |
| C2"-10 | (Cpd. 10) |
| C2"-11 | (Cpd. 11) |
| C2"-12 | (Cpd. 12) |
| C2"-13 | (Cpd. 13) |
| C2"-14 | (Cpd. 14) |

### Exemple C3 : Preparation of a 3 mg/mL lixisenatide solution at pH 4 (Composition C3).

Lixisenatide powder (obtained by peptide synthesis) is dissolved with a 10 mM HCl solution. An acetate buffer solution is then added to give the composition C3. The composition is detailed in Table 2.

### Example C4: Preparation of a stock solution of excipients (matrix) at pH 4 (Composition C4).

A concentrated solution of excipients is prepared via a stepwise addition in an empty container in the following order: m-cresol, acetate buffer and mannitol. Sufficient quantity of water is then added and the pH is adjusted to 4 to obtain Composition C4. This composition is detailed in Table 2.

### Example C5: Preparation of a dilution solution at pH 4 (Composition C5).

A concentrated solution of excipients is prepared via a stepwise addition in an empty container in the following order: BSA (Bovine Serum Albumine), acetate buffer, NaCl and polysorbate 20. Sufficient quantity of water is then added and the pH is adjusted to 4 to obtain Composition C5.

### Exemple C6 : Preparation of a solution of a vehicle (Composition C6).

To a volume VC4 of the composition C4, as prepared in example C4, is added a VC5 of the dilution solution C5, as prepared in example C5 to obtain the final solution C6 which composition is detailed in Table 2.

### Example C7 : Preparation of compositions of short acting amylin RA alone, of compositions of exenatide or lixisenatide or in combinations at pH 4.

The above solutions are mixed to give the compositions detailed in the table below.

In the following table in the excipient column:
- ^{∗}1 means: m-cresol (20 mM), Acetate buffer (20 mM), Mannitol (250 mM)
- ^{∗}2 means : Acetate buffer (20 mM)
- ^{∗}3 means : Acetate buffer (20 mM), BSA (1 mM), NaCl (130 mM), Polysorbate 20 (0.5 mM)
- ^{∗}4 means : m-cresol (3.3 mM), Acetate buffer (20 mM), Mannitol (42 mM), BSA (0.83 mM), NaCl (108 mM), Polysorbate 20 (0.42 mM)

**Table 2: Stock solutions C1 to C4, dilute solution C5, vehicle solution C6, diluted solution of pramlintide C7-1 and C7-2, solutions of exenatide C7-3 and C7-4 and compositions according to the invention C7-5 to C7-32.**

| **Composition** | **Short Acting Amylin RA (concentration in mg/ml)** | **Exenatide or Lixisenatide (concentration in µg/mL)** | **Excipients** |
|---|---|---|---|
| **C1** | - | Exe (250) | ^{∗}1 |
| **C1'** | - | Exe (2000) | ^{∗}2 |
| **C2** | Pramlintide (0.250) | - | ^{∗}1 |
| **C2'** | Pramlintide (10) | - | ^{∗}2 |
| **C3** | - | Lixi (3000) | ^{∗}2 |
| **C4** | - | - | ^{∗}1 |
| **C5** | - | - | ^{∗}3 |
| **C6** | - | - | ^{∗}4 |
| **C7-1** | Pramlintide (0.0042) | - | ^{∗}**4** |
| **C7-2** | Pramlintide (0.042) | - | ^{∗}4 |
| **C7-3** | - | Exenatide (0.42) | ^{∗}1 |
| **C7-4** | - | Exenatide (4.2) | ^{∗}1 |
| **C7-5** | **Cpd 1 (1)** | Exenatide (100) | ^{∗}1 |
| **C7-6** | **Cpd 2 (1)** | Exenatide (100) | ^{∗}1 |
| **C7-7** | **Cpd 3 (1)** | Exenatide (100) | ^{∗}1 |
| **C7-8** | **Cpd 4 (1)** | Exenatide (100) | ^{∗}1 |
| **C7-9** | **Cpd 5 (1)** | Exenatide (100) | ^{∗}1 |
| **C7-10** | **Cpd 6 (1)** | Exenatide (100) | ^{∗}1 |
| **C7-11** | **Cpd 7 (1)** | Exenatide (100) | ^{∗}1 |
| **C7-12** | **Cpd 8 (1)** | Exenatide (100) | ^{∗}1 |
| **C7-13** | **Cpd 9 (1)** | Exenatide (100) | ^{∗}1 |
| **C7-14** | **Cpd 10 (1)** | Exenatide (100) | ^{∗}1 |
| **C7-15** | **Cpd 11 (1)** | Exenatide (100) | ^{∗}1 |
| **C7-16** | **Cpd 12 (1)** | Exenatide (100) | ^{∗}1 |
| **C7-17** | **Cpd 13 (1)** | Exenatide (100) | ^{∗}1 |
| C7-18 | **Cpd 14 (1)** | Exenatide (100) | ^{∗}1 |
| **C7-19** | Cpd 1 (1) | Lixisenatide (250) | ^{∗}1 |
| **C7-20** | **Cpd 2 (1)** | Lixisenatide (250) | ^{∗}1 |
| **C7-21** | **Cpd 3 (1)** | Lixisenatide (250) | ^{∗}1 |
| **C7-22** | **Cpd 4 (1)** | Lixisenatide (250) | ^{∗}1 |
| **C7-23** | **Cpd 5 (1)** | Lixisenatide (250) | ^{∗}1 |
| **C7-24** | **Cpd 6 (1)** | Lixisenatide (250) | ^{∗}1 |
| **C7-25** | **Cpd 7 (1)** | Lixisenatide (250) | ^{∗}1 |
| **C7-26** | **Cpd 8 (1)** | Lixisenatide (250) | ^{∗}1 |
| **C7-27** | **Cpd 9 (1)** | Lixisenatide (250) | ^{∗}1 |
| **C7-28** | **Cpd 10 (1)** | Lixisenatide (250) | ^{∗}1 |
| **C7-29** | **Cpd 11 (1)** | Lixisenatide (250) | ^{∗}1 |
| **C7-30** | **Cpd 12 (1)** | Lixisenatide (250) | ^{∗}1 |
| **C7-31** | **Cpd 13 (1)** | Lixisenatide (250) | ^{∗}1 |
| **C7-32** | **Cpd 14 (1)** | Lixisenatide (250) | ^{∗}1 |

### In vivo studies

### Effects of pramlintide and pramlintide with exenatide compositions on gastric emptying

The aim of this study was to evaluate the effects of pramlintide (compositions C7-1 and C7-2) and co-administration of pramlintide (compositions C7-1 and C7-2) and exenatide (C7-3 and C7-4) on gastro-intestinal transit (gastric emptying) in the mouse.

Co-administration means that the composition of pramlintide and the composition of exenatide were administered separately.

### Example D1: General procedure for in vivo studies of compositions of pramlintide alone and pramlintide co-administered with exenatide

Studies were conducted on naive male Swiss mice (Janvier Lab) as no gender specific effect were expected. Body weight range of 25-32 g were included in the analysis.

Mice were approximately fasted for 18 hours (~T0-18 h). The gastric emptying (i.e. stomach content) was measured via the administration of a suspension of charcoal at T0 (activated charcoal (0.4 ml/mouse, ~0.4mg/mouse; Sigma Reference number C9157), suspended 10% with 2.5% arabic gum in distilled water). The vehicle (as a standard control), the pramlintide composition alone or co-administered with the exenatide composition were administered subcutaneously using Hamilton syringes 20 minutes before charcoal (T0-20 min), then 50 minutes later (T0+50 min), they were sacrificed, and the stomach was removed. The stomach was excised from the abdomen and weighed with its contents (full stomach weight). Afterwards, the stomach was opened, the contents washed out and the weight recorded again (empty stomach weight).

The results are expressed as the variation of gastric content with the administration compositions comprising pramlintide compared with the gastric content with administration the vehicle (weight of gastric content with pramlintide / weight of gastric content with vehicle).

### Example D2: Gastric emptying measurements with compositions comprising pramlintide alone

Measurements of the gastric emptying for compositions C7-2 were obtained according to the general procedure. Five different doses of pramlintide were studied. Each dose was tested on 4 mice. The results of this study are presented in table 3.

**Table 3: Effect of compositions of pramlintide alone on the gastric content**

| **Composition** | **Dose of pramlintide (µg/kg)** | **Concentration of pramlintide (µg/mL)** | **Variability of the stomach content weight compared to the vehicle** |
|---|---|---|---|
| C6 (Vehicle) | -^{∗} | 0 | - |
| C7-2 | 50 | 42 | > 100% |
| C7-2 | 80 | 42 | > 100% |
| C7-2 | 150 | 42 | > 100% |
| C7-2 | 250 | 42 | > 100% |
| C7-2 | 350 | 42 | > 100% |

| | | | |
|---|---|---|---|
| ^{∗} Vehicle administered | | | |

These examples demonstrate that subcutaneous injection of a composition comprising pramlintide alone increases the stomach content weight i.e. slows down the gastric emptying.

### Example D3: Gastric emptying measurements of co-administration of compositions comprising pramlintide or exenatide at different doses compared to compositions of pramlintide alone

Measurements of the gastric emptying for administration of compositions C6 (Vehicle) ; C7-1 and C7-2 (pramlintide alone); and C7-1 or C7-2 (pramlintide alone) co-administered with C7-3 or 7-4 (exenatide alone) were obtained according to the general procedure described in example D1. Two sets of experiments were carried out. Each dose was tested on 8 mice. The results are presented in table 4.

**Table 4: Effect of compositions of pramlintide/exenatide on the gastric content**

| **Composition** | **Pramlintide (µg/kg)** | **Exenatide (µg/kg)** | | **Decrease of the stomach content weight compared to the content weight of the composition of pramlintide alone at the same dose** |
|---|---|---|---|---|
| C6 | -^{∗} | | | - |
| C7-1 | 12.5 | - | | - |
| C7-1 & C7-3 | 12.5 | 0.125 | | > 20 % |
| C7-2 | 25 | - | | - |
| C7-2 & C7-3 | 25 | 0.25 | | > 20 % |
| C6 | -^{∗} | - | | - |
| C7-2 | 25 | - | | |
| C7-2 & C7-4 | 25 | 5 | | > 20 % |
| C7-5 & C7-4 | 25 | 2 | | > 20 % |
| C7-4 & C7-3 | 25 | 0.5 | | > 20 % |

| | | | | |
|---|---|---|---|---|
| ^{∗} Vehicle administered | | | | |

These results show that pramlintide alone increases the gastric content weight i.e., slows down the gastric emptying and that the addition of exenatide to pramlintide decreases the gastric content weight in comparison with the same composition without exenatide i.e., increase the gastric emptying. The decrease of the slowing down of the gastric emptying is more than 20 %.

## Claims

1. Method for the treatment of obesity, overweight, metabolic diseases and/or diabetes, in particular type 2 diabetes, wherein at least one short acting amylin RA and at least one short acting GLP-1 RA are co-administered in a therapeutical scheme that comprises simultaneous or sequential administration.

2. Compositions, at least one of these comprising a short acting amylin RA and doesn't comprise any GLP-1 RA and at least one of these comprising a short acting GLP-1 RA and does not comprise an amylin RA for use for the treatment of obesity, overweight, metabolic diseases and/or diabetes, in particular type 2 diabetes, **characterized in that** the compositions are co-administered in a therapeutical scheme that comprises simultaneous or sequential administration.

3. Composition containing at least one short acting amylin RA in combination with at least one short acting GLP-1 RA for use for treating obesity, overweight, metabolic diseases and/or diabetes, in particular type 2 diabetes.

4. Composition comprising at least one amylin receptor agonist, and at least one short acting GLP-1 receptor agonist such as exenatide and lixisenatide

5. Method or compositions according to any of the preceding claims wherein the short acting amylin RA is chosen amongst the peptides according to Formula 1:
**R₁-Z-R₂** Formula 1
wherein
- R₁ is chosen in the group consisting of hydrogen, C₁₋₄ acyl, benzoyl or C₁₋₄ alkyl;
- R₂ is OH or NHR₃, wherein R₃ is hydrogen or C₁₋₃-alkyl; and
- Z is an amino acid sequence according to formula A1 or B1 or C1 or D1 or E1, and
Formula A1 is the following amino acid sequence as set forth in (SEQ ID NO:3):
Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Xaa25-Xaa26-Xaa27-Xaa28-Xaa29-Thr-Xaa31-Val-Gly-Xaa34-Xaa35-Thr-Xaa37 (SEQ ID NO:3) wherein Xaa represents an amino acid and
wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Asp, Glu, His, Asn, Arg, Gly, Ala, Ser, Lys, Thr and Cys
- Xaa17 is independently selected from His, Arg, Lys and Val;
- Xaa18 is independently selected from Arg, Lys and His;
- Xaa21 is independently selected from Ala, Lys, Gln, Ser, Asn and Pro;
- Xaa22 is independently selected from Glu, Gln, Ser, Thr and Asn;
- Xaa25 is independently selected from Pro and Ala
- Xaa26 is independently selected from Pro, Arg and Ile;
- Xaa27 is independently selected from Pro and Leu;
- Xaa28 is independently selected from Pro and Ser
- Xaa29 is independently selected from Pro and Ser
- Xaa31 is independently selected from Ser, Glu, Asp, Pro and Asn;
- Xaa34 is independently selected from Pro, His, Lys, Arg and Ser;
- Xaa35 is independently selected from His, Arg, Lys, Asp, Glu and Asn;
- Xaa37 is independently selected from Pro and Tyr;
Formula B1 is the following amino acid sequence as set forth in (SEQ ID NO:4): 
X(-1)-X1-X2-X3-X4-X5-X6-X7-X8-X9-X10-X11-X12-X13-X14-X15-X16-X17-X18-X19-X20-X21-
X22-X23-X24-X25-X26-X27-X28-X29-X30-X31-X32-X33-X34-X35-X36 (SEQ IDNO: 4)
 wherein X represents an amino acid and
wherein
- X(-1) is absent or E,
- X1 is absent or selected from the group consisting of E or A,
- X2 is selected from the group consisting of L, A or P,
- X3 is selected from the group consisting of S or P,
- X4 is selected from the group consisting of E, P, K, Q or G ,
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A,
- X33 is selected from the group consisting of P, Y, H, F, L, S, G or A,
- X34 is absent or G,
- X35 is absent or T, and
- X36 is absent or Y,
Formula C1 is the following amino acid sequence as set forth in (SEQ ID NO:5): 
X0-X1-Cys()-X3-Thr-Ala-Thr-Cys()-Ala-Thr-X10-Arg-X12-Ala-X14-Phe-X16-X17-X18-X19-X20-
X21-X22-X23-X24-X25-X26-X27-X28-X29-X30-X31-Val-Gly-Ser-X35-Thr-X37 (SEQ ID NO: 5)
 wherein X represents an amino acid and;
wherein
- X0 is a basic amino acid or is absent;
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X 19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), lle(Me), Dlle(Me), Ala, DAla, Pro and DPro,or is absent;
- X25 is selected from the group consisting of Ala, DAla, Pro and DPro, or is absent;
- X26 is selected from the group consisting of Gly(Me), lle(Me), Dlle(Me), lie and Dlle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, lie, Leu, Thr, Tyr(Me), Gly(Me), Ala(Me), lle(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question;
Formula D1 is the following amino acid sequence as set forth in (SEQ ID NO:6):
Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-lle(Me)-Leu-Ser-Ser-Thr-X31-Val-Gly-Ser-X35-Thr-X37 (SEQ ID NO: 6) wherein X represents an amino acid and
wherein
- X3 is selected from the group consisting of Asn, Gly, Pro and Gln;
- X10 is selected from the group consisting of Gln, Asp and Glu;
- X14 is selected from the group consisting of Asp, His, Asn and Aad;
- X17 is selected from the group consisting of His, Asn, Gln, Glu, Thr, Val, Lys and Aad;
- X19-X20 is selected from the group consisting of Ser-Ser, Val-Val, Ser-Val and Val-Ser, or is absent;
- X31 is selected from the group consisting of Asp, Glu and Asn; X35 is selected from the group consisting of Asp, Glu, Asn, Ser, Phe, Orn, Aad, Gly and Thr; and
- X37 is selected from the group consisting of Pro, Apr and Hyp;
and wherein the compound has at least one residue selected from:
- X3 is Gln;
- X14 is His, Asn or Aad;
- X17 is Asn, Gln, Glu, Thr or Aad;
- X19-X20 is Val-Ser or Ser-Val; and
- X35 is Ser, Phe, Orn, Aad, Gly or Thr;
Formula E1 is the following amino acid sequence as set forth in (SEQ ID NO:7):
X1-X2-X3-X4-X5-X6-X7-Ala-Thr-X10-Arg-Leu-Ala-X14-Phe-Leu-X17-Arg-X19-X20-Phe-Gly(Me)-Ala-lle(Me)-X27-Ser-Ser-Thr-Glu-X32-Gly-Ser-X35-Thr-X37 (SEQ ID NO: 7) wherein X represents an amino acid and
wherein
- X1 is selected from the group consisting of Arg, Lys and Glu;
- X3 is selected from the group consisting of Gly, Gln and Pro;
- X4 is selected from the group consisting of Thr and Glu;
- X5 is selected from the group consisting of Ala and Leu;
- X6 is selected from the group consisting of Thr and Ser;
- X10 is selected from the group consisting of Glu and Gln;
- X14 is selected from the group consisting of Aad, His, Asp, Asn and Arg;
- X17 is selected from the group consisting of Gln, His and Thr;
- X19-X20 is selected from Ser-Ser, Thr-Thr, Ala-Thr, Ala-Ala, Gly-Thr, Gly-Gly and Ala-Asn or is absent;
- X27 is selected from the group consisting of Leu and Pro;
- X32 is selected from the group consisting of Val and Thr;
- X35 is selected from the group consisting of Asn and Ser;
- X37 is selected from the group consisting of Hyp and Pro; and
X2 and X7 are amino acid residues whose side chains together form a lactam bridge;
or a pharmaceutically acceptable salt or solvate thereof.

6. Method or compositions according to any of the preceding claims wherein the short acting amylin RA is chosen amongst the peptides according to Formula 1 :
**R₁-Z-R₂** Formula 1
wherein
- R₁ is hydrogen;
- R₂ is NHR₃, wherein R₃ is hydrogen or C₁₋₃-alkyl; and
- Z is an amino acid sequence according to formula A1 or B1 or C1 or D1 or E1 as previously defined.

7. Method or compositions according to any of the preceding claims wherein the short acting amylin RA is chosen amongst the peptides according to Formula 1 :
**R₁-Z-R₂** Formula 1
wherein
- R₁ is hydrogen;
- R₂ is NHR₃, wherein R₃ is hydrogen, and
- Z is an amino acid sequence according to formula A1 or B1 or C1 or D1 or E1 as previously defined.

8. Method or compositions according to any of the preceding claims wherein the short acting amylin RA is not native human amylin.

9. Method or compositions according to any of the preceding claims wherein the short acting amylin RA is not pramlintide.

10. Method or compositions according to any of the preceding claims wherein the GLP-1 receptor agonist (GLP-1 RA) is chosen amongst lixisenatide and exenatide.

11. Method or compositions according to any of the preceding claims wherein the GLP-1 receptor agonist (GLP-1 RA) is exenatide.

12. Method or compositions according to any of the preceding claims wherein the GLP-1 receptor agonist (GLP-1 RA) is lixisenatide.

13. Method or compositions according to any of the preceding claims wherein the compositions are in the form of an injectable aqueous formulation.

14. Method or compositions according to claim 13, **characterized in that** the pH is ranging from 3.0 to 5.0.

15. Method or compositions according to claim 13, **characterized in that** the pH is ranging from 6.0 to 8.0.

16. Method or compositions according to any of the preceding claims wherein the short acting amylin RA concentration is from 0.3 to 10 mg/ml.

17. Method or compositions according to claim 11, wherein exenatide is at a concentration ranging from 20 to 500 µg/ml.

18. Method or compositions according to claim 12, wherein lixisenatide is at a concentration ranging from 50 to 1250 µg/ml.

19. Method or compositions according to any of the preceding claims, **characterized in that** the ratio short acting amylin RA/ exenatide (weight/weight) is ranging from 2 to 50.

20. Method or compositions according to any of the preceding claims, **characterized in that** the ratio short acting amylin RA/ lixisenatide (weight/weight) is ranging from 0.7 to 20.
